(19) Europäisches Patentamt
European Patent Office
Office européen des brevets

(11) Veröffentlichungsnummer: **0 154 840**
**B1**

(12) **EUROPÄISCHE PATENTSCHRIFT**

(45) Veröffentlichungstag der Patentschrift: **25.01.89**

(21) Anmeldenummer: **85101734.3**

(22) Anmeldetag: **16.02.85**

(51) Int. Cl.⁴: **C 07 D 319/06,** C 07 D 339/08, C 07 D 405/10 // C07D319/00, C07D239/00, C09K19/00

(54) **Flüssigkristalline Verbindungen.**

(30) Priorität: **18.02.84 DE 3405914**

(43) Veröffentlichungstag der Anmeldung: **18.09.85 Patentblatt 85/38**

(45) Bekanntmachung des Hinweises auf die Patenterteilung: **25.01.89 Patentblatt 89/4**

(84) Benannte Vertragsstaaten: **CH DE FR GB LI**

(56) Entgegenhaltungen:
**EP-A-0 087 679**
**EP-A-0 090 183**
**FR-A-2 479 824**
**GB-A-2 115 410**
**US-A-4 313 878**
**US-A-4 424 371**
**US-A-4 490 276**

**Die Akte enthält technische Angaben, die nach dem Eingang der Anmeldung eingereicht wurden und die nicht in dieser Patentschrift enthalten sind.**

(73) Patentinhaber: **Merck Patent Gesellschaft mit beschränkter Haftung, Frankfurter Strasse 250, D-6100 Darmstadt (DE)**

(72) Erfinder: **Krause, Joachim, Dr., Samuel- Morse-Strasse 14, D-6110 Dieburg (DE)**
Erfinder: **Wächtler, Andreas, Dr., Goethestrasse 34, D-6103 Griesheim (DE)**
Erfinder: **Hittich, Reinhard, Dr., Am Kirchberg 11, D-6101 Modautal 1 (DE)**
Erfinder: **Weber, Georg, Wilhelm- Leuschner-Strasse 38, D-6106 Erzhausen (DE)**
Erfinder: **Scheuble, Bernhard, Dr., Am Grenzweg 18, D-6146 Alsbach (DE)**

**EP 0 154 840 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Die Erfindung betrifft Verbindungen der Formel I

$R^1$-$A^1$-$Z^1$-$A^2$-$R^2$ I

worin

| | |
|---|---|
| $R^1$ und $R^2$ | jeweils H, eine Alkyl-, Alkoxy-, Alkanoyl-, Alkanoyloxy- oder Alkoxycarbonyl-Gruppe mit 1 - 10 C-Atomen, F, Cl, Br, CN oder $R^3$-$A^3$-$Z^2$-, |
| $A^1$ | -A-, -$A^4$-A- oder -A-$A^4$-, |
| A | eine 1,3-Dioxan-2,5-diyl- oder eine 1,3-Dithian-2,5-diylgruppe, |
| $A^2$, $A^3$ und $A^4$ | jeweils eine 1,4-Cyclohexylen-, 1,3-Dioxan-2,5-diyl-, 1,3-Dithian-2,5-diyl-, Piperidin-1,4-diyl- oder 1,4-Bicyclo(2,2,2)octylen-Gruppe oder eine unsubstituierte oder durch ein F-Atom oder eine CN-Gruppe substituierte 1,4-Phenylen-, Pyrimidin-2,5-diyl- oder Pyridazin-3,6-diyl-Gruppe, |
| $Z^1$ und $Z^2$ | jeweils -CO-O-, -O-CO-, -$OCH_2$-, -$CH_2O$ oder eine Einfachbindung, und |
| $R^3$ | H, eine Alkyl-, Alkoxy-, Alkanoyl-, Alkanoyloxy- oder Alkoxycarbonyl-Gruppe mit jeweils 1 - 10 C-Atomen, F, Cl, Br oder CN bedeutet, mit der Maßgabe, daß |

(a) mindestens eine Gruppe $A^2$, $A^3$ und/oder $A^4$ eine durch ein F-Atom oder eine CN-Gruppe substituierte 1,4-Phenylen-, Pyrimidin-2,4-diyl oder Pyridazin-3,6-diyl-Gruppe bedeutet und

(b) 1,3-Dioxan-2,5-diyl- und 1,3-Dithian-2,5-diyl-Gruppen nicht in 2-Stellung und Piperidin-1,4-diyl-Gruppen nicht in 1-Stellung durch Alkoxy, Alkanoyl, Alkanoyloxy, Alkoxycarbonyl, F, Cl, Br, CN oder $R^3$-$A^3$-$Z^2$ substituiert sind,

sowie Verbindungen der Formeln

$R^1$-Dio-Phe-$CH_2CH_2$-Phe-$R^2$ I4
$R^1$-Dio-Phe-$CH_2CH_2$-Cy-$R^2$ I5
$R^1$-Dio-Phe-$CH_2CH_2$-Phe-Cy-$R^3$ I6
$R^1$-Dio-Phe-$CH_2CH_2$-Phe-Dio-$R^3$ I7
$R^1$-Dio-$CH_2CH_2$-Phe-Phe-$R^2$ I17
$R^1$-Dio-$CH_2CH_2$-Phe-Phe-Cy-$R^2$ I18
$R^1$-Dio-$CH_2CH_2$-Phe-Cy-$R^2$ I19

worin

| | |
|---|---|
| Dio | eine 1,3-Dioxan-2,5-diylgruppe |
| Phe | eine 1,4-Phenylengruppe |
| Cy | eine 1,4-Cyclohexylengruppe |
| und | |
| $R^1$, $R^2$ und $R^3$ | jeweils H, eine Alkyl-, Alkoxy-, Alkanoyl-, Alkanoyloxy- oder Alkoxycarbonyl-Gruppe mit 1- 10 C-atomen, F, Cl, Br oder CN |

bedeutet und mindestens eine Gruppe Phe eine durch ein F-Atom oder eine CN-Gruppe substituierte 1,4-Phenylen-Gruppe bedeutet,

sowie die Säureadditionssalze der basischen unter diesen Verbindungen.

Der Einfachheit halber bedeuten im folgenden Cy eine 1,4-Cyclohexylengruppe, Dio eine 1,3-Dioxan-2,5-diylgruppe, Dit eine 1,3-Dithian-2,5-diylgruppe, Bi eine Bicyclo-(2,2,2)-octylengruppe, Pip eine Piperidin-1,4-diylgruppe, Phe eine 1,4-Phenylengruppe, Pyr eine Pyrimidin-2,5-diylgruppe und Pyn eine Pyridazin-3,6-diylgruppe, wobei Phe und/oder Pyr und/oder Pyn unsubstituiert oder durch ein F-Atom oder eine CN-Gruppe substituiert sein können, wobei mindestens eine dieser Gruppen in den Verbindungen nach Anspruch 1 lateral substituiert ist.

Die Verbindungen nach Anspruch 1 können als Komponenten flüssigkristalliner Phasen verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Ähnliche Dioxanverbindungen und deren Verwendung als Mischungskomponente für flüssigkristalline Mischungen sind bereits bekannt. So sind in GB-A-2 115 410, EP-A-87 679, FR-A-2 479 824 und US-A-4 313 878 Verbindungen mit lateral unsubstituierten Ringen beschrieben. In der EP-A-90 183 werden mehrfach lateral durch Halogen substituierte, einen Dioxanring enthaltende Aromaten beschrieben. Die US-A-4 424 371 enthalten Verbindungen, die einfach lateral durch Chlor substituiert sind. Alle dort genannten Substanzen weisen Nachteile verschiedenster Art auf: zu hohe Schmelzpunkte, zu niedrige Klärpunkte insbesondere bei den Polyhalogenverbindungen, zu geringe Stabilität gegenüber der Einwirkung von Wärme, Licht oder elektrischen Feldern oder eine zu hohe Temperaturabhängigkeit der Schwellenspannung.

Der Erfindung lag somit die Aufgabe zugrunde, neue stabile flüssigkristalline oder mesogene Verbindungen

aufzufinden, die als Komponenten flüssigkristalliner Phasen geeignet sind und die genannten Nachteile nicht oder nur im geringen Maße aufweisen. Diese Aufgabe wurde durch die Bereitstellung der Verbindungen nach Anspruch 1 gelöst.

Es wurde gefunden, daß die Verbindungen nach Anspruch 1 als Komponenten flüssigkristalliner Phasen vorzüglich geeignet sind. Insbesondere sind mit ihrer Hilfe stabile flüssigkristalline Phasen mit breitem Mesophasenbereich und vergleichsweise niedriger Viskosität herstellbar.

Mit der Bereitstellung der Verbindungen nach Anspruch 1 wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen nach Anspruch 1 besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen nach Anspruch 1 flüssigkristallinen Basismaterialien aus anderen Verbindungsklassen zugesetzt werden, um beispielsweise die dielektrische und/oder optische Anisotropie eines solchen Dielektrikums zu beeinflussen. Die Verbindungen nach Anspruch 1 eignen sich ferner als Zwischenprodukte zur Herstellung anderer Substanzen, die sich als Bestandteile flüssigkristalliner Dielektrika verwenden lassen.

Die Verbindungen nach Anspruch 1 sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen nach Anspruch 1 sowie ein Verfahren zur Herstellung von Verbindungen nach Anspruch 1, dadurch gekennzeichnet, daß man einen entsprechenden Aldehyd mit einem entsprechenden Diol bzw. Dithiol umsetzt,

oder daß man eine Verbindung, die sonst einer Verbindung nach Anspruch 1 entspricht, aber an Stelle von H-Atomen eine oder mehrere reduzierbare Gruppen und/oder C-C-Bindungen enthält, mit einem reduzierenden Mittel behandelt,

oder daß man zur Herstellung von Estern nach Anspruch 1 (worin $Z^1$ und/oder $Z^2$ -CO-O- oder -O-CO-bedeuten und/oder $R^1$ und/oder $R^2$ und/oder $R^3$ eine Carboxylgruppe enthalten) eine entsprechende Carbonsäure oder eines ihrer reaktionsfähigen Derivate mit einem entsprechenden Alkohol oder einem seiner reaktionsfähigen Derivate umsetzt,

oder daß man zur Herstellung von Nitrilen nach Anspruch 1 (worin $R^1$ und/oder $R^2$ und/oder $R^3$ CN bedeuten und/oder worin $A^2$ und/oder $A^3$ und/oder $A^4$ durch eine CN-Gruppe substituiert sind) ein entsprechendes Carbonsäureamid dehydratisiert oder ein entsprechendes Carbonsäurehalogenid mit Sulfamid umsetzt,

oder daß man zur Herstellung von Ethern nach Anspruch 1 (worin $R^1$ und/oder $R^2$ eine Alkoxygruppe bedeuten und/oder $Z^1$ und/oder $Z^2$ eine $-OCH_2-$ oder $-CH_2O-$Gruppe sind) eine entsprechende Hydroxyverbindung verethert,

und/oder daß man gegebenenfalls eine entsprechende Chlor- oder Bromverbindung (worin $R^1$ und/oder $R^2$ und/oder $R^3$ Cl oder Br bedeuten und/oder worin $A^2$ und/oder $A^3$ und/oder $A^4$ durch ein Chlor- oder Bromatom substituiert sind) mit einem Cyanid umsetzt,

und/oder daß man gegebenenfalls eine Base nach Anspruch 1 durch Behandeln mit einer Säure in eines ihrer Säureadditionssalze umwandelt,

oder daß man gegebenenfalls eine Verbindung nach Anspruch 1 aus einem ihrer Säureadditionssalze durch Behandeln mit einer Base freisetzt.

Weiterhin ist Gegenstand der Erfindung die Verwendung der Verbindungen nach Anspruch 1 als Komponenten flüssigkristalliner Phasen. Gegenstand der Erfindung sind ferner flüssigkristalline Phasen mit einem Gehalt an mindestens einer Verbindung nach Anspruch 1 sowie Flüssigkristall-Anzeigeelemente, die derartige Phasen enthalten.

Vor- und nachstehend haben $R^1$, $R^2$, $R^3$, $A^1$, $A^2$, $A^3$, $A^4$, A, $Z^1$ und $Z^2$, die angegebene Bedeutung, sofern nicht ausdrücklich etwas anderes vermerkt ist.

Falls $R^1$ und $R^2$ die Bedeutung $R^3$-$A^3$-$Z^2$- haben, umfassen Verbindungen nach Anspruch 1 Verbindungen in denen $R^3$, $A^3$ und/oder $Z^2$ gleich oder voneinander verschieden sind.

Die Verbindungen nach Anspruch 1 umfassen dementsprechend Verbindungen mit zwei Ringen der Teilformeln Ia und Ib:

$R^1$-A-$Z^1$-$A^2$-$R^2$ Ia
$R^1$-A-$A^2$-$R^2$ Ib

Verbindungen mit drei Ringen der Teilformeln Ic bis Il:

$R^1$-$A^4$-A-$Z^1$-$A^2$-$R^2$ Ic
$R^1$-A-$A^4$-$Z^1$-$A^2$-$R^2$ Id
$R^1$-A-$Z^1$-$A^2$-$Z^2$-$A^3$-$R^3$ Ie
$R^3$-$A^3$-$Z^2$-A-$Z^1$-$A^2$-$R^2$ If
$R^1$-A-$Z^1$-$A^2$-$A^3$-$R^2$ Ig
$R^1$-A-$A^2$-$Z^2$-$A^3$-$R^3$ Ih
$R^3$-$A^3$-$Z^2$-A-$A^2$-$R^2$ Ii

$R^3$-$A^3$-A-$Z^1$-$A^2$-$R^2$ Ij
$R^1$-$A^4$-A-$A^2$-$R^2$ Ik
$R^1$-A-$A^4$-$A^2$-$R^2$ Il,

Verbindungen mit vier Ringen der Teilformeln Im bis Iff:

$R^3$-$A^3$-$Z^2$-A-$Z^1$-$A^2$-$Z^2$-$A^3$-$R^3$ Im
$R^3$-$A^3$-A-$Z^1$-$A^2$-$A^3$-$R^3$ In
$R^3$-$A^3$-$Z^2$-A-$A^2$-$A^3$-$R^3$ Io
$R^3$-$A^3$-A-$A^2$-$Z^2$-$A^3$-$R^3$ Ip
$R^1$-$A^4$-A-$Z^1$-$A^2$-$Z^2$-$A^3$-$R^3$ Iq
$R^3$-$A^3$-$Z^2$-$A^4$-A-$Z^1$-$A^2$-$R^2$ Ir
$R^1$-A-$A^4$-$Z^1$-$A^2$-$Z^2$-$A^3$-$R^3$ Is
$R^3$-$A^3$-$Z^2$-A-$A^4$-$Z^1$-$A^2$-$R^2$ It
$R^1$-$A^4$-A-$A^2$-$A^3$-$R^3$ Iu
$R^1$-A-$A^4$-$A^2$-$A^3$-$R^3$ Iv
$R^1$-$A^4$-A-$A^2$-$Z^2$-$A^3$-$R^3$ Iw
$R^1$-A-$A^4$-$A^2$-$Z^2$-$A^3$-$R^3$ Ix
$R^1$-$A^4$-A-$Z^1$-$A^2$-$A^3$-$R^3$ Iy
$R^1$-A-$A^4$-$Z^1$-$A^2$-$A^3$-$R^3$ Iz
$R^3$-$A^3$-$A^4$-A-$A^2$-$R^2$ Iaa
$R^3$-$A^3$-A-$A^4$-$A^2$-$R^2$ Ibb
$R^3$-$A^3$-$A^4$-A-$Z^1$-$A^2$-$R^2$ Icc
$R^3$-$A^3$-A-$A^4$-$Z^1$-$A^2$-$R^2$ Idd
$R^3$-$A^3$-$Z^2$-$A^4$-A-$A^2$-$R^2$ Iee
$R^3$-$A^3$-$Z^2$-A-$A^4$-$A^2$-$R^2$ Iff,

sowie Verbindungen mit fünf Ringen der Teilformeln Igg bis Ill:

$R^3$-$A^3$-$Z^2$-$A^4$-A-$Z^1$-$A^2$-$Z^2$-$A^3$-$R^3$ Igg
$R^3$-$A^3$-$Z^2$-A-$A^4$-$Z^1$-$A^2$-$Z^2$-$A^3$-$R^3$ Ihh
$R^3$-$A^3$-$A^4$-A-$A^2$-$A^3$-$R^3$ Iii
$R^3$-$A^3$-A-$A^4$-$A^2$-$A^3$-$R^3$ Ijj
$R^3$-$A^3$-$A^4$-A-$Z^1$-$A^2$-$A^3$-$R^3$ Ikk
$R^3$-$A^3$-A-$A^4$-$Z^1$-$A^2$-$A^3$-$R^3$ Ill.

In den Verbindungen der vor- und nachstehenden Formeln bedeuten $R^1$ und $R^2$ vorzugsweise Alkyl, ferner Alkoxy.

Weiterhin bevorzugt sind Verbindungen der vor- und nachstehenden Formeln, in denen einer der Reste $R^1$ und $R^2$ CN, F oder Cl bedeutet. Vorzugsweise enthalten solche Verbindungen jedoch nur einen Substituenten aus der Reihe Cl und CN.

$A^2$, $A^3$ und $A^4$ sind bevorzugt Cy, Phe oder Pyr, ferner bevorzugt Dio oder Dit; bevorzugt enthält die Verbindung nach Anspruch 1 nicht mehr als einen der Reste Dio, Dit, Pip, Bi, Pyn oder Pyr.

Substituierte ungesättigte Gruppen $A^2$, $A^3$ und/oder $A^4$ umfassen solche der Struktur 1,

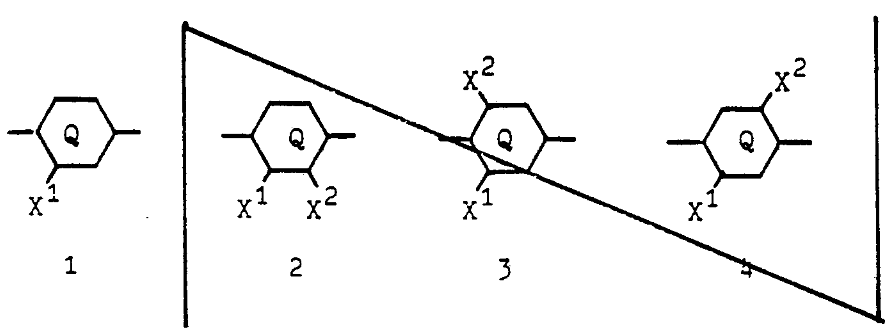

1 2 3 4

worin der Ring Q eine 1,4-Phenylengruppe, eine Pyrimidin-2,5-diylgruppe oder eine Pyridazin-3,6-diylgruppe bedeutet und $X^1$ jeweils die Bedeutung F und/oder CN hat.

Bevorzugte substituierte Gruppen $A^2$, $A^3$ und/oder $A^4$ sind diejenigen der Struktur 1, wobei Q vorzugsweise eine 1,4-Phenylengruppe ist. Eine besonders bevorzugte Gruppe $X^1$ ist F.

A ist vorzugsweise eine 1,3-Dioxan-2,5-diylgruppe.

$Z^1$ und $Z^2$ sind bevorzugt Einfachbindungen, in zweiter Linie bevorzugt -CO-O- oder -O-CO-Gruppen.

$R^3$ ist vorzugsweise eine Alkylgruppe mit 1 - 10 C-Atomen oder CN.

Falls $R^1$ und/oder $R^2$ Alkylreste und/oder Alkoxyreste bedeuten, so können sie geradkettig oder verzweigt sein. Vorzugsweise sind sie geradkettig, haben 2, 3, 4, 5, 6 oder 7 C-Atome und bedeuten demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Methoxy, Octoxy, Nonoxy oder Decoxy.

Verbindungen nach Anspruch 1 mit verzweigten Flügelgruppen $R^1$ bzw. $R^2$ oder $R^3$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind.

Verzweigte Gruppen dieser Art enthalten in der Regel nicht mehr als eine Kettenverzweigung. Bevorzugte verzweigte Reste $R^1$ und $R^2$ sind Isopropyl, 2-Butyl (= 1-Methylpropyl), Isobutyl (= 2-Methylpropyl), 2-Methylbutyl, Isopentyl (= 3-Methylbutyl),. 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Anspruch 1 umfaßt sowohl die Racemate der genannten Verbindungen als auch die optischen Antipoden sowie deren Gemische.

Unter den Verbindungen nach Anspruch 1 sowie Ia bis III sind diejenigen bevorzugt, in denen mindestens einer der darin enthaltenen Reste eine der angegebenen bevorzugten Bedeutungen hat. Besonders bevorzugte kleinere Gruppen von Verbindungen sind diejenigen der Formeln I1 bis I19:

| | |
|---|---|
| $R^1$-Dio-COO-Phe-$R^2$ | I1 |
| $R^1$-Dio-OCO-Phe-$R^2$ | I2 |
| $R^1$-Dio-Phe-OCO-Phe-$R^2$ | I3 |
| $R^1$-Dio-Phe-$CH_2CH_2$-Phe-$R^2$ | I4 |
| $R^1$-Dio-Phe-$CH_2CH_2$-Cy-$R^2$ | I5 |
| $R^1$-Dio-Phe-$CH_2CH_2$-Phe-Cy-$R^3$ | I6 |
| $R^1$-Dio-Phe-$CH_2CH_2$-Phe-Dio-$R^3$ | I7 |
| $R^1$-Dio-Phe-$R^2$ | I8 |
| $R^1$-Dio-Phe-Phe-$R^2$ | I9 |
| $R^1$-Dio-Phe-Phe-Cy-$R^3$ | I10 |
| $R^1$-Dio-Phe-Phe-Dio-$R^3$ | I11 |
| $R^1$-Dit-Phe-$R^2$ | I12 |
| $R^1$-Dio-Cy-Phe-$R^2$ | I13 |
| $R^1$-Cy-Dio-Phe-$R^2$ | I14 |
| $R^1$-Dio-Pyr-$R^2$ | I15 |
| $R^1$-Dit-Pyr-$R^2$ | I16 |
| $R^1$-Dio-$CH_2CH_2$-Phe-Phe-$R^2$ | I17 |
| $R^1$-Dio-$CH_2CH_2$-Phe-Phe-Cy-$R^2$ | I18 |
| $R^1$-Dio-$CH_2CH_2$-Phe-Cy-$R^2$ | I19 |

Weiterhin bevorzugt sind Verbindungen der Formel I20,

| | |
|---|---|
| $R^1$-Dio-$A^4$-CO-O-Q-CN | I20 |

worin Q 3-Fluor-1,4-phenylen bedeutet und $A^4$ Phe oder Cy ist.

In den Verbindungen nach Anspruch 1 sind diejenigen Stereoisomeren bevorzugt, in denen die Ringe Cy, Dio, Dit und/oder Pip trans-1,4-disubstituiert sind. Diejenigen der vorstehend genannten Formeln, die eine oder mehrere Gruppen Dio, Dit, Pip und/oder Pyr enthalten, umschließen jeweils die beiden möglichen 2,5-(Dio, Dit, Pyr) bzw. 1,4-Stellungsisomeren (Pip).

Besonders bevorzugt sind Verbindungen nach Anspruch 1 worin $R^1$ und $R^2$ jeweils geradkettige oder höchstens einfach verzweigte Alkylgruppen oder Alkoxygruppen mit 1 - 10 C-Atomen, F, Cl, Br oder CN bedeuten.

Die Verbindungen nach Anspruch 1 werden nach an sich bekannten Methoden hergestellt, wie sie in der Literatur (z. B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen nach Anspruch 1 umsetzt.

So können die Verbindungen nach Anspruch 1 hergestellt werden, indem man einen entsprechenden Aldehyd oder eines seiner reaktionsfähigen Derivate mit einem entsprechenden Diol bzw. Dithiol umsetzt.

Der Fachmann kann durch Routinemethoden entsprechende Synthesemethoden aus dem Stand der Technik entnehmen (z. B. DE-OS-2 944 905; DE-OS-3 227 916; DD-160 061; US-4 322 354; US-4 298 528; US-4 323 504; US-4 200 580; US-4 313 878).

Die Reaktionspartner oder ihre reaktionsfähigen Derivate werden zweckmäßig in Gegenwart eines inerten Lösungsmittels wie Benzol oder Toluol und/oder eines Katalysators, z. B. einer starken Säure wie Schwefelsäure, Benzol- oder p-Toluolsulfonsäure, oder einer Lewis-Säure wie Aluminiumchlorid bei

Temperaturen zwischen etwa 20° und etwa 150°, vorzugsweise zwischen 80° und 120° miteinander umgesetzt.

Als reaktionsfähige Derivate der Ausgangsstoffe eignen sich in erster Linie Acetale.

Die genannten Aldehyde und 1,3-Diole bzw. 1,3-Dithiole sowie ihre reaktionsfähigen Derivate sind zum Teil bekannt, zum Teil können sie ohne Schwierigkeiten nach Standardverfahren der organischen Chemie aus literaturbekannten Verbindungen hergestellt werden. Beipielsweise sind die Aldehyde durch Oxydation entsprechender Alkohole oder durch Reduktion entsprechender Carbonsäuren oder ihrer Derivate, die Diole durch Reduktion entsprechender Diester und die Dithiole durch Umsetzung entsprechender Dihalogenide mit NaSH erhältlich.

Verbindungen nach Anspruch 1 sind weiterhin erhältlich, indem man eine Verbindung, die sonst einer Verbindung nach Anspruch 1 entspricht, aber an stelle von H-Atomen eine oder mehrere reduzierbare Gruppen und/oder C-C-Bindungen enthält, reduziert.

Als reduzierbare Gruppen kommen vorzugsweise Carbonylgruppen in Betracht, insbesondere Ketogruppen, ferner z. B. freie oder veresterte Hydroxygruppen oder aromatisch gebundene Halogenatome. Bevorzugte Ausgangsstoffe für die Reduktion entsprechen Verbindungen nach Anspruch 1 können aber an Stelle eines Cyclohexanrings einen Cyclohexenring oder Cyclohexanonring und/oder an Stelle einer $-CH_2CH_2-$Gruppe eine $-CH=CH-$Gruppe und/oder an Stelle einer $-CH_2-$Gruppe eine -CO-Gruppe und/oder an Stelle eines H-Atoms eine freie oder eine funktionell (z. B. in Form ihres p-Toluolsulfonats) abgewandelte OH-Gruppe enthalten.

Die Reduktion kann z. B. erfolgen durch katalytische Hydrierung bei Temperaturen zwischen etwa 0° und etwa 200° sowie Drucken zwischen etwa 1 und 200 bar in einem inerten Lösungsmittel, z. B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z. B. $PtO_2$, PdO), auf einem Träger (z. B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120°) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperaturen zwischen etwa 100 und 200°) zu den entsprechenden Verbindungen nach Anspruch 1 die Alkylgruppen und/oder $-CH_2CH_2-$Brücken enthalten, reduziert werden.

Weiterhin sind Reduktionen mit komplexen Hydriden möglich. Beispielsweise können Arylsulfonyloxygruppen mit $LiAlH_4$ reduktiv entfernt werden, insbesondere p-Toluolsulfonyloxymethylgruppen zu Methylgruppen reduziert werden, zweckmäßig in einem inerten Lösungsmittel wie Diethylether oder THF bei Temperaturen zwischen etwa 0 und 100°. Doppelbindungen können (auch in Gegenwart von CN-Gruppen!) mit $NaBH_4$ oder Tributylzinnhydrid in Methanol hydriert werden.

Ester nach Anspruch 1 können auch durch Veresterung entsprechender Carbonsäuren (oder ihrer reaktionsfähigen Derivate) mit Alkoholen bzw. Phenolen (oder ihren reaktionsfähigen Derivaten) erhalten werden.

Als reaktionsfähige Derivate der genannten Carbonsäuren eignen sich insbesondere die Säurehalogenide, vor allem die Chloride und Bromide, ferner die Anhydride, z. B. auch gemischte Anhydride, Azide oder Ester, insbesondere Alkylester mit 1 - 4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls wie Na oder K, in Betracht.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z. B. Pyridin, Chinolin oder Triethylamin als Lösungsmittel für die Veresterung angewandt werden. Die Veresterung kann auch in Abwesenheit eines Lösungsmittels, z. B. durch einfaches Erhitzen der Komponenten in Gegenwart von Natriumacetat, durchgeführt werden. Die Reaktionstemperatur liegt gewöhnlich zwischen -50° und +250°, vorzugsweise zwischen -20° und +80°. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z. B. durch Behandlung mit ethanolischer Natron-oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmäßig bei Temperaturen zwischen etwa -25° und +20°.

Zur Herstellung von Nitrilen nach Anspruch 1 (worin $R^1$ und/oder $R^2$ CN bedeuten und/oder worin $A^2$, $A^3$ und/oder $A^4$ durch eine CN-Gruppe substituiert ist) können entsprechende Säureamide, z. B. solche, in denen an Stelle des Restes X eine $CONH_2$-Gruppe steht, dehydratisiert werden. Die Amide sind z. B. aus entsprechenden Estern oder Säurehalogeniden durch Umsetzung mit Ammoniak erhältlich. Als wasserabspaltende Mittel eignen sich beispielsweise anorganische Säurechloride wie $SOCl_2$, $PCl_3$, $PCl_5$, $POCl_3$, $SO_2Cl_2$, $COCl_2$, ferner $P_2O_5$, $P_2S_5$, $AlCl_3$ (z. B. als Doppelverbindung mit NaCl), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten; als Lösungsmittel kommen z. B. Basen wie Pyridin oder Triethylamin, aromatische Kohlenwasserstoffe wie Benzol, Toluol oder Xylol oder Amide wie DMF in Betracht.

Zur Herstellung der vorstehend genannten Nitrile nach Anspruch 1 kann man auch entsprechende Säurehalogenide, vorzugsweise die Chloride, mit Sulfamid umsetzen, zweckmäßig in einem inerten Lösungsmittel wie Tetramethylensulfon bei Temperaturen zwischen etwa 80° und 150°, vorzugsweise bei 120°. Nach üblicher Aufarbeitung kann man direkt die Nitrile isolieren.

Ether nach Anspruch 1 (worin $R^1$ und/oder $R^2$ und/oder $R^3$ eine Alkoxygruppe bedeuten und/oder worin $Z^1$ und/oder $Z^2$ eine $-OCH_2-$ oder eine $-CH_2O$-Gruppe ist) sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z. B. durch Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie Aceton, 1,2-Dimethoxyethan, DMF oder Dimethylsulfoxid oder auch einem Überschuß an wäßriger oder wäßrig-alkoholischer NaOH oder KOH bei Temperaturen zwischen etwa 20° und 100°.

Zur Herstellung von Nitrilen nach Anspruch 1 (worin $R^1$ und/oder $R^2$ CN bedeuten und/oder worin $A^2$, $A^3$ und/oder $A^4$ durch eine CN-Gruppe substituiert ist) können auch entsprechende Chlor- oder Bromverbindungen (worin $R^1$ und/oder $R^2$ Cl oder Br bedeuten und/oder worin A durch ein Cl- oder Br-Atom substituiert ist) mit einem Cyanid umgesetzt werden, zweckmäßig mit einem Metallcyanid wie NaCN, KCN oder $Cu_2(CN)_2$, z. B. in Gegenwart von Pyridin in einem inerten Lösungsmittel wie DMF oder N-Methylpyrrolidon bei Temperaturen zwischen 20° und 200°.

Eine Base nach Anspruch 1 kann mit einer Säure in das zugehörige Säureadditionssalz übergeführt werden. Für diese Umsetzung können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z. B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -di-sulfonsäuren, Laurylschwefelsäure.

Umgekehrt ist es möglich, aus einem Säureadditionssalz einer Verbindung nach Anspruch 1 die Base nach Anspruch 1 durch Behandeln mit einer Base freizusetzen, z. B. mit einer starken anorganischen Base wie KOH oder NaOH.

Die erfindungsgemäßen flüssigkristallinen Phasen bestehen aus 2 bis 20, vorzugsweise 3 bis 15 Komponenten, darunter mindestens einer Verbindung nach Anspruch 1. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Biscyclohexylbiphenyle, Phenyl- oder Cyclohexylpyrimidine, Phenyl- oder Cyclohexyldioxane, Phenyl- oder Cyclohexyldithiane, 1,2-Bis-phenylethane, 1,2-Bis-cyclohexylethane, 1-Phenyl-2-cyclohexylethane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel II charakterisieren,

R'-L-G-E-R'' II

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| G | -CH=CH- | -N(O)=N- |
| | -CH=CY- | -CH=N(O)- |
| | -C≡C- | $-CH_2-CH_2-$ |
| | -CO-O- | $-CH_2-O-$ |
| | -CO-S- | $-CH_2-S-$ |
| | -CH=N- | -COO-Phe-COO- |

oder eine C-C-Einfachbindung, Y Halogen, vorzugsweise Chlor, oder -CN, und R' und R'' Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten.

Bei den meisten dieser Verbindungen sind R' und R'' voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle diese Substanzen sind nach literaturbekannten Methoden herstellbar.

Die erfindungsgemäßen flüssigkristallinen Phasen enthalten etwa 0,1 bis 99, vorzugsweise 10 bis 95 %, einer oder mehrerer Verbindungen nach Anspruch 1. Weiterhin bevorzugt sind flüssigkristalline Phasen, die 0,1 - 50, insbesondere 0,5 - 30 % einer oder mehrerer Verbindungen nach Anspruch 1 enthalten.

Die Herstellung der erfindungsgemäßen flüssigkristallinen Phasen erfolgt in an sich üblicher Weise. In der Regel werden die Komponenten ineinander gelöst, zweckmäßig bei erhöhter Temperatur.

Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammoniumtetraphenylboranat oder Komplexsalze von Kronenethern (vgl. z. B. I. Haller et al., Mol.Cryst.Liq.Cryst. Band 24, seiten 249 - 258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z. B. in den DE-OS-2 209 127, 2 240 864, 2 321 632, 2 338 281, 2 450 088, 2 637 430, 2 853 728 und 2 902 177 beschrieben.

Die folgenden Beispiele sollen die Erfindung erläutern, ohne sie zu begrenzen. F. = Schmelzpunkt, K. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

**Beispiel 1**

6,2 g Terephthalaldehydsäure-2-cyano-4-butylphenylester (erhältlich durch Umsetzung von Terephthalaldehydsäurechlorid mit 3-Cyano-4-n-butylphenol in Gegenwart von Pyridin) und 3,3 g 2-Pentylpropandiol-1,3 werden in Toluol gelöst, mit einer Spatelspitze p-Toluolsulfonsäure versetzt und am Wasserabscheider erhitzt, bis kein Wasser mehr gebildet wird. Nach dem Abkühlen wird das Reaktionsgemisch mit Bicarbonatlösung säurefrei gewaschen, über Natriumsulfat getrocknet und wie üblich aufgearbeitet. Man erhält 4-(5-Pentyl-1,3-dioxan-2-yl)-benzoesäure-(3-cyano-4-butylphenylester), F. 47°, K. 121°.

Analog werden hergestellt:

4-(5-Pentyl-1,3-dioxan-2-yl)-benzoesäure-(3-cyano-4-ethylphenylester)
4-(5-Pentyl-1,3-dioxan-2-yl)-benzoesäure-(3-cyano-4-propylphenylester)
4-(5-Pentyl-1,3-dioxan-2-yl)-benzoesäure-(3-cyano-4-pentylphenylester)
4-(5-Pentyl-1,3-dioxan-2-yl)-benzoesäure-(3-cyano-4-heptylphenylester)
4-(5-Pentyl-1,3-dioxan-2-yl)-benzoesäure-(3-cyano-4-ethoxyphenylester)
4-(5-Pentyl-1,3-dioxan-2-yl)-benzoesäure-(3-cyano-4-propoxyphenylester)
4-(5-Pentyl-1,3-dioxan-2-yl)-benzoesäure-(3-cyano-4-butoxyphenylester)
4-(5-Pentyl-1,3-dioxan-2-yl)-benzoesäure-(3-cyano-4-pentoxyphenylester)
4-(5-Pentyl-1,3-dioxan-2-yl)-benzoesäure-(3-cyano-4-hexoxyphenylester
4-(5-Pentyl-1,3-dioxan-2-yl)-benzoesäure-(3-cyano-4-heptoxyphenylester
4-(5-Pentyl-1,3-dioxan-2-yl)-benzoesäure-(2-fluor-4-ethylphenylester)
4-(5-Pentyl-1,3-dioxan-2-yl)-benzoesäure-(2-fluor-4-propylphenylester)
4-(5-Pentyl-1,3-dioxan-2-yl)-benzoesäure-(2-fluor-4-pentylphenylester)
4-(5-Pentyl-1,3-dioxan-2-yl)-benzoesäure-(2-fluor-4-heptylphenylester)
4-(5-Pentyl-1,3-dioxan-2-yl)-benzoesäure-(2-fluor-4-ethoxyphenylester)
4-(5-Pentyl-1,3-dioxan-2-yl)-benzoesäure-(2-fluor-4-propoxyphenylester)

4-(5-Pentyl-1,3-dioxan-2-yl)-benzoesäure-(2-fluor-4-butoxyphenylester)
4-(5-Pentyl-1,3-dioxan-2-yl)-benzoesäure-(2-fluor-4-pentoxyphenylester)
4-(5-Pentyl-1,3-dioxan-2-yl)-benzoesäure-(2-fluor-4-hexoxyphenylester)
4-(5-Pentyl-1,3-dioxan-2-yl)-benzoesäure-(2-fluor-4-heptoxyphenylester)
4-(5-Pentyl-1,3-dioxan-2-yl)-cylohexancarbonsäure-(2-cyan-4-ethylphenylester)
4-(5-Pentyl-1,3-dioxan-2-yl)-cylohexancarbonsäure-(2-cyan-4-propylphenylester)
4-(5-Pentyl-1,3-dioxan-2-yl)-cylohexancarbonsäure-(2-cyan-4-pentylphenylester)
4-(5-Pentyl-1,3-dioxan-2-yl)-cylohexancarbonsäure-(2-cyan-4-heptylphenylester)
4-(5-Pentyl-1,3-dioxan-2-yl)-cylohexancarbonsäure-(2-cyan-4-ethoxyphenylester)
4-(5-Pentyl-1,3-dioxan-2-yl)-cylohexancarbonsäure-(2-cyan-4-propoxyphenylester)
4-(5-Pentyl-1,3-dioxan-2-yl)-cylohexancarbonsäure-(2-cyan-4-butoxyphenylester)
4-(5-Pentyl-1,3-dioxan-2-yl)-cylohexancarbonsäure-(2-cyan-4-pentoxyphenylester)
4-(5-Pentyl-1,3-dioxan-2-yl)-cylohexancarbonsäure-(2-cyan-4-hexoxyphenylester)
4-(5-Pentyl-1,3-dioxan-2-yl)-cylohexancarbonsäure-(2-cyan-4-heptoxyphenylester)
4-(5-Pentyl-1,3-dioxan-2-yl)-cyolohexancarbonsäure-(2-fluor-4-ethylphenylester)
4-(5-Pentyl-1,3-dioxan-2-yl)-cyclohexancarbonsäure-(2-fluor-4-ethylpropylester)
4-(5-Pentyl-1,3-dioxan-2-yl)-cyclohexancarbonsäure-(2-fluor-4-pentylphenylester)
4-(5-Pentyl-1,3-dioxan-2-yl)-cyclohexancarbonsäure-(2-fluor-4-heptylphenylester)
4-(5-Pentyl-1,3-dioxan-2-yl)-cyclohexancarbonsäure-(2-fluor-4-ethoxyphenylester)
4-(5-Pentyl-1,3-dioxan-2-yl)-cyclohexancarbonsäure-(2-fluor-4-propoxyphenylester)
4-(5-Pentyl-1,3-dioxan-2-yl)-cyclohexancarbonsäure-(2-fluor-4-butoxyphenylester)
4-(5-Pentyl-1,3-dioxan-2-yl)-cyclohexancarbonsäure-(2-fluor-4-pentoxyphenylester)
4-(5-Pentyl-1,3-dioxan-2-yl)-cyclohexancarbonsäure-(2-fluor-4-hexoxyphenylester)
4-(5-Pentyl-1,3-dioxan-2-yl)-cyclohexancarbonsäure-(2-fluor-4-heptoxyphenylester)

Analog werden hergestellt durch Veresterung der entsprechenden Carbonsäuren mit 3-Fluor-4-cyanophenol:

4-(5-Ethyl-1,3-dioxan-2-yl)-benzoesäure-(3-fluor-4-cyanophenylester)
4-(5-Propyl-1,3-dioxan-2-yl)-benzoesäure-(3-fluor-4-cyanophenylester)
4-(5-Butyl-1,3-dioxan-2-yl)-benzoesäure-(3-fluor-4-cyanophenylester)
4-(5-Pentyl-1,3-dioxan-2-yl)-benzoesäure-(3-fluor-4-cyanophenylester)
4-(5-Hexyl-1,3-dioxan-2-yl)-benzoesäure-(3-fluor-4-cyanophenylester)
4-(5-Heptyl-1,3-dioxan-2-yl)-benzoesäure-(3-fluor-4-cyanophenylester)
4-(5-Octyl-1,3-dioxan-2-yl)-benzoesäure-(3-fluor-4-cyanophenylester)
4-(5-Nonyl-1,3-dioxan-2-yl)-benzoesäure-(3-fluor-4-cyanophenylester)
4-(5-Decyl-1,3-dioxan-2-yl)-benzoesäure-(3-fluor-4-cyanophenylester)
4-(5-Ethyl-1,3-dioxan-2-yl)-cyclohexancarbonsäure-(3-fluor-4-cyanophenylester)
4-(5-Propyl-1,3-dioxan-2-yl)-cyclohexancarbonsäure-(3-fluor-4-cyanophenylester)
4-(5-Butyl-1,3-dioxan-2-yl)-cyclohexancarbonsäure-(3-fluor-4-cyanophenylester)
4-(5-Pentyl-1,3-dioxan-2-yl)-cyclohexancarbonsäure-(3-fluor-4-cyanophenylester)
4-(5-Hexyl-1,3-dioxan-2-yl)-cyclohexancarbonsäure-(3-fluor-4-cyanophenylester)
4-(5-Heptyl-1,3-dioxan-2-yl)-cyclohexancarbonsäure-(3-fluor-4-cyanophenylester)
4-(5-Octyl-1,3-dioxan-2-yl)-cyclohexancarbonsäure-(3-fluor-4-cyanophenylester)
4-(5-Nonyl-1,3-dioxan-2-yl)-cyclohexancarbonsäure-(3-fluor-4-cyanophenylester)
4-(5-Decyl-1,3-dioxan-2-yl)-cyclohexancarbonsäure-(3-fluor-4-cyanophenylester)

**Beispiel 2**

6,5 g 4-(trans-4-Propylcylohexyl)-2-fluorbiphenyl-4'-carbaldehyd (erhältlich durch Bromierung von 4-(trans-4-Propylcylohexyl)-2-fluorbiphenyl mit Brom in Gegenwart katalytischer Mengen Jod und Umsetzung der aus der Bromverbindung erhaltenen magnesium-organischen Verbindungen mit N-Formylpiperidin) und 3,5 g 2-Heptylpropandiol-1,3 werden zusammen mit 1 g stark saurem Kationenaustauscher in 100 ml Toluol 8 Stdn. am Wasserabscheider erhitzt. Anschließend wird der Katalysator abfiltriert und das Toluol abdestilliert. Nach üblicher Aufarbeitung erhält man 4-(trans-4-Propylcylohexyl)-2-fluor-4'-(5-heptyl-1,3-dioxan-2-yl)-biphenyl.

Analog werden hergestellt:

4-(trans-4-Ethyl-cyclohexyl)-2-fluor-4'-(5-heptyl-1,3-dioxan-2-yl)-biphenyl
4-(trans-4-Butyl-cyclohexyl)-2-fluor-4'-(5-heptyl-1,3-dioxan-2-yl)-biphenyl
4-(trans-4-Pentyl-cyclohexyl)-2-fluor-4'-(5-heptyl-1,3-dioxan-2-yl)-biphenyl
4-(trans-4-Heptyl-cyclohexyl)-2-fluor-4'-(5-heptyl-1,3-dioxan-2-yl)-biphenyl
4-(trans-4-Ethylcyclohexyl)-2-fluor-4'-(5-pentyl-1,3-dioxan-2-yl)-biphenyl
4-(trans-4-Propylcyclohexyl)-2-fluor-4'-(5-pentyl-1,3-dioxan-2-yl)-biphenyl

4-(trans-4-Butylcyclohexyl)-2-fluor-4'-(5-pentyl-1,3-dioxan-2-yl)-biphenyl
4-(trans-4-Pentylcyclohexyl)-2-fluor-4'-(5-pentyl-1,3-dioxan-2-yl)-biphenyl
4-(trans-4-Heptylcyclohexyl)-2-fluor-4'-(5-pentyl-1,3-dioxan-2-yl)-biphenyl
4-(trans-4-Ethyl-cyclohexyl)-2'-fluor-4'-(5-pentyl-1,3-dioxan-2-yl)-biphenyl
4-(trans-4-Propyl-cyclohexyl)-2'-fluor-4'-(5-pentyl-1,3-dioxan-2-yl)-biphenyl
4-(trans-4-Butyl-cyclohexyl)-2'-fluor-4'-(5-pentyl-1,3-dioxan-2-yl)-biphenyl
4-(trans-4-Pentyl-cyclohexyl)-2'-fluor-4'-(5-pentyl-1,3-dioxan-2-yl)-biphenyl
4-(trans-4-Heptyl-cyclohexyl)-2'-fluor-4'-(5-pentyl-1,3-dioxan-2-yl)-biphenyl
4-(trans-4-Ethylcyclohexyl)-2-fluor-4'-(5-pentyl-1,3-dithian-2-yl)-biphenyl
4-(trans-4-Propylcyclohexyl)-2-fluor-4'-(5-pentyl-1,3-dithian-2-yl)-biphenyl
4-(trans-4-Butylcyclohexyl)-2-fluor-4'-(5-pentyl-1,3-dithian-2-yl)-biphenyl
4-(trans-4-Pentylcyclohexyl)-2-fluor-4'-(5-pentyl-1,3-dithian-2-yl)-biphenyl
4-(trans-4-Heptylcyclohexyl)-2-fluor-4'-(5-pentyl-1,3-dithian-2-yl)-biphenyl
4-(5-Ethyl-1,3-dioxan-2-yl)-2-fluor-4'-(5-pentyl-1,3-dioxan-2-yl)-biphenyl
4-(5-Propyl-1,3-dioxan-2-yl)-2-fluor-4'-(5-pentyl-1,3-dioxan-2-yl)-biphenyl
4-(5-Butyl-1,3-dioxan-2-yl)-2-fluor-4'-(5-pentyl-1,3-dioxan-2-yl)-biphenyl
4-(5-Hexyl-1,3-dioxan-2-yl)-2-fluor-4'-(5-pentyl-1,3-dioxan-2-yl)-biphenyl
4-(5-Heptyl-1,3-dioxan-2-yl)-2-fluor-4'-(5-pentyl-1,3-dioxan-2-yl)-biphenyl
4-(trans-4-Ethylcyclohexyl)-2-fluor-4'-(5-propyl-1,3-dioxan-2-yl)-biphenyl
4-(trans-4-Propylcyclohexyl)-2-fluor-4'-(5-propyl-1,3-dioxan-2-yl)-biphenyl
4-(trans-4-Butylcyclohexyl)-2-fluor-4'-(5-propyl-1,3-dioxan-2-yl)-biphenyl
4-(trans-4-Pentylcyclohexyl)-2-fluor-4'-(5-propyl-1,3-dioxan-2-yl)-biphenyl, F. 83°, K. 272°
4-(trans-4-Heptylcyclohexyl)-2-fluor-4'-(5-propyl-1,3-dioxan-2-yl)-biphenyl
4-(5-Pentyl-1,3-dioxan-2-yl)-2-cyan-1-ethyl-benzol
4-(5-Pentyl-1,3-dioxan-2-yl)-2-cyan-1-propyl-benzol
4-(5-Pentyl-1,3-dioxan-2-yl)-2-cyan-1-butyl-benzol
4-(5-Pentyl-1,3-dioxan-2-yl)-2-cyan-1-pentyl-benzol
4-(5-Pentyl-1,3-dioxan-2-yl)-2-cyan-1-heptyl-benzol
4-(5-Propyl-1,3-dioxan-2-yl)-2-fluor-1-ethyl-benzol
4-(5-Propyl-1,3-dioxan-2-yl)-2-fluor-1-propyl-benzol
4-(5-Propyl-1,3-dioxan-2-yl)-2-fluor-1-butyl-benzol
4-(5-Propyl-1,3-dioxan-2-yl)-2-fluor-1-pentyl-benzol
4-(5-Propyl-1,3-dioxan-2-yl)-2-fluor-1-heptyl-benzol
4-(5-Propyl-1,3-dithian-2-yl)-2-fluor-1-ethyl-benzol
4-(5-Propyl-1,3-dithian-2-yl)-2-fluor-1-propyl-benzol
4-(5-Propyl-1,3-dithian-2-yl)-2-fluor-1-butyl-benzol
4-(5-Propyl-1,3-dithian-2-yl)-2-fluor-1-pentyl-benzol
4-(5-Propyl-1,3-dithian-2-yl)-2-fluor-1-heptyl-benzol
4-(5-Pentyl-1,3-dithian-2-yl)-2-cyan-1-ethyl-benzol
4-(5-Pentyl-1,3-dithian-2-yl)-2-cyan-1-propyl-benzol
4-(5-Pentyl-1,3-dithian-2-yl)-2-cyan-1-butyl-benzol
4-(5-Pentyl-1,3-dithian-2-yl)-2-cyan-1-pentyl-benzol
4-(5-Pentyl-1,3-dithian-2-yl)-2-cyan-1-heptyl-benzol
4-(5-Pentyl-1,3-dioxan-2-yl)-2-fluor-4'-ethyl-biphenyl
4-(5-Pentyl-1,3-dioxan-2-yl)-2-fluor-4'-propyl-biphenyl
4-(5-Pentyl-1,3-dioxan-2-yl)-2-fluor-4'-butyl-biphenyl
4-(5-Pentyl-1,3-dioxan-2-yl)-2-fluor-4'-pentyl-biphenyl
4-(5-Pentyl-1,3-dioxan-2-yl)-2-fluor-4'-heptyl-biphenyl
4-(5-Heptyl-1,3-dioxan-2-yl)-2'-fluor-4'-ethyl-biphenyl
4-(5-Heptyl-1,3-dioxan-2-yl)-2'-fluor-4'-propyl-biphenyl
4-(5-Heptyl-1,3-dioxan-2-yl)-2'-fluor-4'-butyl-biphenyl
4-(5-Heptyl-1,3-dioxan-2-yl)-2'-fluor-4'-pentyl-biphenyl
4-(5-Heptyl-1,3-dioxan-2-yl)-2'-fluor-4'-heptyl-biphenyl
4-(5-Butyl-1,3-dithian-2-yl)-2'-fluor-4'-ethyl-biphenyl
4-(5-Butyl-1,3-dithian-2-yl)-2'-fluor-4'-propyl-biphenyl
4-(5-Butyl-1,3-dithian-2-yl)-2'-fluor-4'-butyl-biphenyl
4-(5-Butyl-1,3-dithian-2-yl)-2'-fluor-4'-pentyl-biphenyl
4-(5-Butyl-1,3-dithian-2-yl)-2'-fluor-4'-heptyl-biphenyl
trans-1-(5-Pentyl-1,3-dioxan-2-yl)-4-(4-ethyl-3-fluor-phenyl)-cylohexan
trans-1-(5-Pentyl-1,3-dioxan-2-yl)-4-(4-propyl-3-fluor-phenyl)-cylohexan
trans-1-(5-Pentyl-1,3-dioxan-2-yl)-4-(4-butyl-3-fluor-phenyl)-cylohexan
trans-1-(5-Pentyl-1,3-dioxan-2-yl)-4-(4-pentyl-3-fluor-phenyl)-cylohexan
trans-1-(5-Pentyl-1,3-dioxan-2-yl)-4-(4-heptyl-3-fluor-phenyl)-cylohexan
trans-1-(5-Pentyl-1,3-dioxan-2-yl)-4-(4-ethyl-2-fluor-phenyl)-cylohexan
trans-1-(5-Pentyl-1,3-dioxan-2-yl)-4-(4-propyl-2-fluor-phenyl)-cylohexan

trans-1-(5-Pentyl-1,3-dioxan-2-yl)-4-(4-butyl-2-fluor-phenyl)-cylohexan
trans-1-(5-Pentyl-1,3-dioxan-2-yl)-4-(4-pentyl-2-fluor-phenyl)-cylohexan
trans-1-(5-Pentyl-1,3-dioxan-2-yl)-4-(4-heptyl-2-fluor-phenyl)-cylohexan
2-(4-Ethyl-2-fluor-phenyl)-5-(trans-4-pentylcylohexyl)-1,3-dioxan
2-(4-Propyl-2-fluor-phenyl)-5-(trans-4-pentylcylohexyl)-1,3-dioxan
2-(4-Butyl-2-fluor-phenyl)-5-(trans-4-pentylcylohexyl)-1,3-dioxan
2-(4-Pentyl-2-fluor-phenyl)-5-(trans-4-pentylcylohexyl)-1,3-dioxan
2-(4-Heptyl-2-fluor-phenyl)-5-(trans-4-pentylcylohexyl)-1,3-dioxan
2-(4-Ethyl-3-fluor-phenyl)-5-(trans-4-pentylcyclohexyl)-1,3-dioxan
2-(4-Propyl-3-fluor-phenyl)-5-(trans-4-pentylcyclohexyl)-1,3-dioxan
2-(4-Butyl-3-fluor-phenyl)-5-(trans-4-pentylcyclohexyl)-1,3-dioxan
2-(4-Pentyl-3-fluor-phenyl)-5-(trans-4-pentylcyclohexyl)-1,3-dioxan
2-(4-Heptyl-3-fluor-phenyl)-5-(trans-4-pentylcyclohexyl)-1,3-dioxan
2-(4-Ethyl-3-fluor-phenyl)-5-(trans-4-pentylcyclohexyl)-1,3-dithian
2-(4-Propyl-3-fluor-phenyl)-5-(trans-4-pentylcyclohexyl)-1,3-dithian
2-(4-Butyl-3-fluor-phenyl)-5-(trans-4-pentylcyclohexyl)-1,3-dithian
2-(4-Pentyl-3-fluor-phenyl)-5-(trans-4-pentylcyclohexyl)-1,3-dithian
2-(4-Heptyl-3-fluor-phenyl)-5-(trans-4-pentylcyclohexyl)-1,3-dithian
5-(5-Pentyl-1,3-dioxan-2-yl)-4-fluor-2-cyan-pyrimidin
5-(5-Pentyl-1,3-dioxan-2-yl)-4-fluor-2-ethyl-pyrimidin
5-(5-Pentyl-1,3-dioxan-2-yl)-4-fluor-2-propyl-pyrimidin
5-(5-Pentyl-1,3-dioxan-2-yl)-4-fluor-2-butyl-pyrimidin
5-(5-Pentyl-1,3-dioxan-2-yl)-4-fluor-2-pentyl-pyrimidin
5-(5-Pentyl-1,3-dioxan-2-yl)-4-fluor-2-heptyl-pyrimidin
5-(5-Pentyl-1,3-dithian-2-yl)-4-fluor-2-cyan-pyrimidin
5-(5-Pentyl-1,3-dithian-2-yl)-4-fluor-2-ethyl-pyrimidin
5-(5-Pentyl-1,3-dithian-2-yl)-4-fluor-2-propyl-pyrimidin
5-(5-Pentyl-1,3-dithian-2-yl)-4-fluor-2-butyl-pyrimidin
5-(5-Pentyl-1,3-dithian-2-yl)-4-fluor-2-pentyl-pyrimidin
5-(5-Pentyl-1,3-dithian-2-yl)-4-fluor-2-heptyl-pyrimidin
5-[4-(5-Pentyl-1,3-dioxan-2-yl)-phenyl]-4-fluor-2-cyan-pyrimidin
5-[4-(5-Pentyl-1,3-dioxan-2-yl)-phenyl]-4-fluor-2-ethyl-pyrimidin
5-[4-(5-Pentyl-1,3-dioxan-2-yl)-phenyl]-4-fluor-2-propyl-pyrimidin
5-[4-(5-Pentyl-1,3-dioxan-2-yl)-phenyl]-4-fluor-2-butyl-pyrimidin
5-[4-(5-Pentyl-1,3-dioxan-2-yl)-phenyl]-4-fluor-2-pentyl-pyrimidin
5-[4-(5-Pentyl-1,3-dioxan-2-yl)-phenyl]-4-fluor-2-heptyl-pyrimidin
5-[4-(5-Propyl-1,3-dioxan-2-yl)-phenyl]-4-fluor-2-cyan-pyrimidin
5-[4-(5-Propyl-1,3-dioxan-2-yl)-phenyl]-4-fluor-2-ethyl-pyrimidin
5-[4-(5-Propyl-1,3-dioxan-2-yl)-phenyl]-4-fluor-2-propyl-pyrimidin
5-[4-(5-Propyl-1,3-dioxan-2-yl)-phenyl]-4-fluor-2-butyl-pyrimidin
5-[4-(5-Propyl-1,3-dioxan-2-yl)-phenyl]-4-fluor-2-pentyl-pyrimidin
5-[4-(5-Propyl-1,3-dioxan-2-yl)-phenyl]-4-fluor-2-heptyl-pyrimidin
2-[4-(5-Pentyl-1,3-dioxan-2-yl)-phenyl]-4-fluor-5-cyan-pyrimidin
2-[4-(5-Pentyl-1,3-dioxan-2-yl)-phenyl]-4-fluor-5-ethyl-pyrimidin
2-[4-(5-Pentyl-1,3-dioxan-2-yl)-phenyl]-4-fluor-5-propyl-pyrimidin
2-[4-(5-Pentyl-1,3-dioxan-2-yl)-phenyl]-4-fluor-5-butyl-pyrimidin
2-[4-(5-Pentyl-1,3-dioxan-2-yl)-phenyl]-4-fluor-5-pentyl-pyrimidin
2-[4-(5-Pentyl-1,3-dioxan-2-yl)-phenyl]-4-fluor-5-heptyl-pyrimidin
2-[trans-4-(5-Pentyl-1,3-dioxan-2-yl)-cyclohexyl]-4-fluor-5-cyan-pyrimidin
2-[trans-4-(5-Pentyl-1,3-dioxan-2-yl)-cyclohexyl]-4-fluor-5-ethyl-pyrimidin
2-[trans-4-(5-Pentyl-1,3-dioxan-2-yl)-cyclohexyl]-4-fluor-5-propyl-pyrimidin
2-[trans-4-(5-Pentyl-1,3-dioxan-2-yl)-cyclohexyl]-4-fluor-5-butyl-pyrimidin
2-[trans-4-(5-Pentyl-1,3-dioxan-2-yl)-cyclohexyl]-4-fluor-5-pentyl-pyrimidin
2-[trans-4-(5-Pentyl-1,3-dioxan-2-yl)-cyclohexyl]-4-fluor-5-heptyl-pyrimidin
2-[4-(5-Pentyl-1,3-dioxan-2-yl)-biphenyl-4'-yl]-4-fluor-5-cyan-pyrimidin
2-[4-(5-Pentyl-1,3-dioxan-2-yl)-biphenyl-4'-yl]-4-fluor-5-ethyl-pyrimidin
2-[4-(5-Pentyl-1,3-dioxan-2-yl)-biphenyl-4'-yl]-4-fluor-5-propyl-pyrimidin
2-[4-(5-Pentyl-1,3-dioxan-2-yl)-biphenyl-4'-yl]-4-fluor-5-butyl-pyrimidin
2-[4-(5-Pentyl-1,3-dioxan-2-yl)-biphenyl-4'-yl]-4-fluor-5-pentyl-pyrimidin
2-[4-(5-Pentyl-1,3-dioxan-2-yl)-biphenyl-4'-yl]-4-fluor-5-heptyl-pyrimidin
2-[4-(5-Butyl-1,3-dithian-2-yl)-biphenyl-4'-yl]-4-fluor-5-cyan-pyrimidin
2-[4-(5-Butyl-1,3-dithian-2-yl)-biphenyl-4'-yl]-4-fluor-5-ethyl-pyrimidin
2-[4-(5-Butyl-1,3-dithian-2-yl)-biphenyl-4'-yl]-4-fluor-5-propyl-pyrimidin
2-[4-(5-Butyl-1,3-dithian-2-yl)-biphenyl-4'-yl]-4-fluor-5-butyl-pyrimidin
2-[4-(5-Butyl-1,3-dithian-2-yl)-biphenyl-4'-yl]-4-fluor-5-pentyl-pyrimidin

2-[4-(5-Butyl-1,3-dithian-2-yl)-biphenyl-4'-yl]-4-fluor-5-heptyl-pyrimidin

**Beispiel 3**

6 g 3-(4'-Pentyl-2-fluorbiphenyl-4-yl)-propionaldehyd (erhältlich durch Umsetzung von 4-(2-Fluor-4'-pentyl-biphenylyl)-magnesiumbromid mit Ethylenoxid und anschließende Oxidation des entstandenen Alkohols mit Pyridiniumchlorochromat) und 3,5 g 2-Heptylpropandiol-1,3 werden mit einer Spatelspitze p-Toluolsulfonsäure in 100 ml Toluol am Wasserabscheider erhitzt, bis sich kein Wasser mehr bildet. Nach dem Abkühlen wäscht man mit Bicarbonatlösung und Wasser neutral, trocknet über Natriumsulfat und destilliert das Toluol ab. Nach üblicher Aufarbeitung erhält man 1-(4'-Pentyl-2-fluorbiphenyl-4-yl)-2-(5-heptyl-1,3-dioxan-2-yl)-ethan.

Analog werden hergestellt:

1-(4'-Ethyl-2-fluorbiphenyl-4-yl)-2-(5-heptyl-1,3-dioxan-2-yl)-ethan
1-(4'-Propyl-2-fluorbiphenyl-4-yl)-2-(5-heptyl-1,3-dioxan-2-yl)-ethan
1-(4'-Butyl-2-fluorbiphenyl-4-yl)-2-(5-heptyl-1,3-dioxan-2-yl)-ethan
1-(4'-Pentyl-2-fluorbiphenyl-4-yl)-2-(5-heptyl-1,3-dioxan-2-yl)-ethan
1-(4'-Heptyl-2-fluorbiphenyl-4-yl)-2-(5-heptyl-1,3-dioxan-2-yl)-ethan
1-(4'-Cyan-2-fluorbiphenyl-4-yl)-2-(5-heptyl-1,3-dioxan-2-yl)-ethan
1-(4'-Ethoxy-2-fluorbiphenyl-4-yl)-2-(5-heptyl-1,3-dioxan-2-yl)-ethan
1-(4'-Propoxy-2-fluorbiphenyl-4-yl)-2-(5-heptyl-1,3-dioxan-2-yl)-ethan
1-(4'-Butoxy-2-fluorbiphenyl-4-yl)-2-(5-heptyl-1,3-dioxan-2-yl)-ethan
1-(4'-Pentoxy-2-fluorbiphenyl-4-yl)-2-(5-heptyl-1,3-dioxan-2-yl)-ethan
1-(4'-Heptoxy-2-fluorbiphenyl-4-yl)-2-(5-heptyl-1,3-dioxan-2-yl)-ethan
1-(4'-Nonoxy-2-fluorbiphenyl-4-yl)-2-(5-heptyl-1,3-dioxan-2-yl)-ethan
1-(4'-Ethyl-2'-fluorbiphenyl-4-yl)-2-(5-heptyl-1,3-dioxan-2-yl)-ethan
1-(4'-Propyl-2'-fluorbiphenyl-4-yl)-2-(5-heptyl-1,3-dioxan-2-yl)-ethan
1-(4'-Butyl-2'-fluorbiphenyl-4-yl)-2-(5-heptyl-1,3-dioxan-2-yl)-ethan
1-(4'-Pentyl-2'-fluorbiphenyl-4-yl)-2-(5-heptyl-1,3-dioxan-2-yl)-ethan
1-(4'-Heptyl-2'-fluorbiphenyl-4-yl)-2-(5-heptyl-1,3-dioxan-2-yl)-ethan
1-(4'-Cyan-2'-fluorbiphenyl-4-yl)-2-(5-heptyl-1,3-dioxan-2-yl)-ethan
1-(4'-Ethoxy-2'-fluorbiphenyl-4-yl)-2-(5-heptyl-1,3-dioxan-2-yl)-ethan
1-(4'-Propoxy-2'-fluorbiphenyl-4-yl)-2-(5-heptyl-1,3-dioxan-2-yl)-ethan
1-(4'-Butoxy-2'-fluorbiphenyl-4-yl)-2-(5-heptyl-1,3-dioxan-2-yl)-ethan
1-(4'-Pentoxy-2'-fluorbiphenyl-4-yl)-2-(5-heptyl-1,3-dioxan-2-yl)-ethan
1-(4'-Heptoxy-2'-fluorbiphenyl-4-yl)-2-(5-heptyl-1,3-dioxan-2-yl)-ethan
1-(4'-Nonoxy-2'-fluorbiphenyl-4-yl)-2-(5-heptyl-1,3-dioxan-2-yl)-ethan
1-(4'-Ethyl-2'-fluorbiphenyl-4-yl)-2-(5-pentyl-1,3-dioxan-2-yl)-ethan
1-(4'-Propyl-2'-fluorbiphenyl-4-yl)-2-(5-pentyl-1,3-dioxan-2-yl)-ethan
1-(4'-Butyl-2'-fluorbiphenyl-4-yl)-2-(5-pentyl-1,3-dioxan-2-yl)-ethan
1-(4'-Heptyl-2'-fluorbiphenyl-4-yl)-2-(5-pentyl-1,3-dioxan-2-yl)-ethan
1-(4'-Cyan-2'-fluorbiphenyl-4-yl)-2-(5-pentyl-1,3-dioxan-2-yl)-ethan
1-(4'-Ethoxy-2'-fluorbiphenyl-4-yl)-2-(5-pentyl-1,3-dioxan-2-yl)-ethan
1-(4'-Propoxy-2'-fluorbiphenyl-4-yl)-2-(5-pentyl-1,3-dioxan-2-yl)-ethan
1-(4'-Butoxy-2'-fluorbiphenyl-4-yl)-2-(5-pentyl-1,3-dioxan-2-yl)-ethan
1-(4'-Pentoxy-2'-fluorbiphenyl-4-yl)-2-(5-pentyl-1,3-dioxan-2-yl)-ethan
1-(4'-Heptoxy-2'-fluorbiphenyl-4-yl)-2-(5-pentyl-1,3-dioxan-2-yl)-ethan
1-(4'-Nonoxy-2'-fluorbiphenyl-4-yl)-2-(5-pentyl-1,3-dioxan-2-yl)-ethan
1-(4'-Ethyl-2'-fluorbiphenyl-4-yl)-2-(5-propyl-1,3-dioxan-2-yl)-ethan
1-(4'-Propyl-2'-fluorbiphenyl-4-yl)-2-(5-propyl-1,3-dioxan-2-yl)-ethan
1-(4'-Butyl-2'-fluorbiphenyl-4-yl)-2-(5-propyl-1,3-dioxan-2-yl)-ethan
1-(4'-Pentyl-2'-fluorbiphenyl-4-yl)-2-(5-propyl-1,3-dioxan-2-yl)-ethan, F. 36°, K. 98°
1-(4'-Heptyl-2'-fluorbiphenyl-4-yl)-2-(5-propyl-1,3-dioxan-2-yl)-ethan
1-(4'-Cyan-2'-fluorbiphenyl-4-yl)-2-(5-propyl-1,3-dioxan-2-yl)-ethan
1-(4'-Ethoxy-2'-fluorbiphenyl-4-yl)-2-(5-propyl-1,3-dioxan-2-yl)-ethan
1-(4'-Propoxy-2'-fluorbiphenyl-4-yl)-2-(5-propyl-1,3-dioxan-2-yl)-ethan
1-(4'-Butoxy-2'-fluorbiphenyl-4-yl)-2-(5-propyl-1,3-dioxan-2-yl)-ethan
1-(4'-Pentoxy-2'-fluorbiphenyl-4-yl)-2-(5-propyl-1,3-dioxan-2-yl)-ethan
1-(4'-Heptoxy-2'-fluorbiphenyl-4-yl)-2-(5-propyl-1,3-dioxan-2-yl)-ethan
1-(4'-Nonoxy-2'-fluorbiphenyl-4-yl)-2-(5-propyl-1,3-dioxan-2-yl)-ethan
1-(4'-Ethyl-2'-fluorbiphenyl-4-yl)-2-(5-butyl-1,3-di-thian-2-yl)-ethan
1-(4'-Propyl-2'-fluorbiphenyl-4-yl)-2-(5-butyl-1,3-di-thian-2-yl)-ethan
1-(4'-Butyl-2'-fluorbiphenyl-4-yl)-2-(5-butyl-1,3-di-thian-2-yl)-ethan

1-(4'-Pentyl-2'-fluorbiphenyl-4-yl)-2-(5-butyl-1,3-dithian-2-yl)-ethan
1-(4'-Heptyl-2'-fluorbiphenyl-4-yl)-2-(5-butyl-1,3-dithian-2-yl)-ethan
1-(4'-Cyan-2'-fluorbiphenyl-4-yl)-2-(5-butyl-1,3-di-thian-2-yl)-ethan
1-(4'-Ethoxy-2'-fluorbiphenyl-4-yl)-2-(5-butyl-1,3-di-thian-2-yl)-ethan
1-(4'-Propoxy-2'-fluorbiphenyl-4-yl)-2-(5-butyl-1,3-di-thian-2-yl)-ethan
1-(4'-Butoxy-2'-fluorbiphenyl-4-yl)-2-(5-butyl-1,3-di-thian-2-yl)-ethan
1-(4'-Pentoxy-2'-fluorbiphenyl-4-yl)-2-(5-butyl-1,3-di-thian-2-yl)-ethan
1-(4'-Heptoxy-2'-fluorbiphenyl-4-yl)-2-(5-butyl-1,3-di-thian-2-yl)-ethan
1-(4'-Nonoxy-2'-fluorbiphenyl-4-yl)-2-(5-butyl-1,3-di-thian-2-yl)-ethan
1-(4'-(trans-4-Ethylcylohexyl)-2'-fluorbiphenyl-4-yl]-2-(5-pentyl-1,3-dioxan-2-yl)-ethan
1-[4'-(trans-4-Propylcylohexyl)-2'-fluorbiphenyl-4-yl]-2-(5-pentyl-1,3-dioxan-2-yl)-ethan
1-[4'-(trans-4-Butylcylohexyl)-2'-fluorbiphenyl-4-yl]-2-(5-pentyl-1,3-dioxan-2-yl)-ethan
1-[4'-(trans-4-Pentylcylohexyl)-2'-fluorbiphenyl-4-yl]-2-(5-pentyl-1,3-dioxan-2-yl)-ethan
1-[4'-(trans-4-Heptylcylohexyl)-2'-fluorbiphenyl-4-yl]-2-(5-pentyl-1,3-dioxan-2-yl)-ethan
1-[4-(trans-4-Ethylcylohexyl)-2-fluor-phenyl]-2-(5-heptyl-1,3-dioxan-2-yl)-ethan
1-[4-(trans-4-Propylcylohexyl)-2-fluor-phenyl]-2-(5-heptyl-1,3-dioxan-2-yl)-ethan
1-[4-(trans-4-Butylcylohexyl)-2-fluor-phenyl]-2-(5-heptyl-1,3-dioxan-2-yl)-ethan
1-[4-(trans-4-Pentylcylohexyl)-2-fluor-phenyl]-2-(5-heptyl-1,3-dioxan-2-yl)-ethan
1-[4-(trans-4-Heptylcylohexyl)-2-fluor-phenyl]-2-(5-heptyl-1,3-dioxan-2-yl)-ethan
1-[4-(trans-4-Ethylcylohexyl)-3-fluor-phenyl]-2-(5-pentyl-1,3-dioxan-2-yl)-ethan
1-[4-(trans-4-Propylcylohexyl)-3-fluor-phenyl]-2-(5-pentyl-1,3-dioxan-2-yl)-ethan
1-[4-(trans-4-Butylcylohexyl)-3-fluor-phenyl]-2-(5-pentyl-1,3-dioxan-2-yl)-ethan
1-[4-(trans-4-Pentylcylohexyl)-3-fluor-phenyl]-2-(5-pentyl-1,3-dioxan-2-yl)-ethan
1-[4-(trans-4-Heptylcylohexyl)-3-fluor-phenyl]-2-(5-pentyl-1,3-dioxan-2-yl)-ethan
1-(4'-Cyan-3'-fluorbiphenyl-4-yl)-2-(5-methyl-1,3-dioxan-2-yl)-ethan
1-(4'-Cyan-3'-fluorbiphenyl-4-yl)-2-(5-ethyl-1,3-dioxan-2-yl)-ethan
1-(4'-Cyan-3'-fluorbiphenyl-4-yl)-2-(5-propyl-1,3-dioxan-2-yl)-ethan
1-(4'-Cyan-3'-fluorbiphenyl-4-yl)-2-(5-butyl-1,3-dioxan-2-yl)-ethan
1-(4'-Cyan-3'-fluorbiphenyl-4-yl)-2-(5-pentyl-1,3-dioxan-2-yl)-ethan
1-(4'-Cyan-3'-fluorbiphenyl-4-yl)-2-(5-hexyl-1,3-dioxan-2-yl)-ethan
1-(4'-Cyan-3'-fluorbiphenyl-4-yl)-2-(5-heptyl-1,3-dioxan-2-yl)-ethan
1-(4'-Cyan-3'-fluorbiphenyl-4-yl)-2-(5-octyl-1,3-dioxan-2-yl)-ethan

**Beispiel 4**

Zu 0,5 g 2-Hexyl-1,3-dioxan-5-carbonsäure (FR-2 521 581-A), 10 ml Ether und 0,2 ml Pyridin werden bei 0° 0,3 g Thionylchlorid gegeben. Nach zwei stunden wird vom Pyridinhydrochlorid abgesaugt, das Filtrat mit 2 ml Pyridin und 0,75 g 5-Hydroxy-2-propoxybenzonitril versetzt. Nach 3 Stunden wird wie üblich aufgearbeitet. Man erhält 2-n-Hexyl-1,3-dioxan-5-carbonsäure-(3-cyan-4-propoxy-phenylester).

Analog werden hergestellt:

2-Hexyl-1,3-dioxan-5-carbonsäure-(3-cyan-4-ethoxyphenylester)
2-Hexyl-1,3-dioxan-5-carbonsäure-(3-cyan-4-butoxyphenylester)
2-Hexyl-1,3-dioxan-5-carbonsäure-(3-cyan-4-pentoxyphenylester)
2-Hexyl-1,3-dioxan-5-carbonsäure-(3-cyan-4-heptoxyphenylester)
2-Hexyl-1,3-dioxan-5-carbonsäure-(3-cyan-4-cyanphenylester)
2-Pentyl-1,3-dioxan-5-carbonsäure-(2-fluor-4-ethylphenylester)
2-Pentyl-1,3-dioxan-5-carbonsäure-(2-fluor-4-propyl-phenylester)
2-Pentyl-1,3-dioxan-5-carbonsäure-(2-fluor-4-butyl-phenylester)
2-Pentyl-1,3-dioxan-5-carbonsäure-(2-fluor-4-pentyl-phenylester)
2-Pentyl-1,3-dioxan-5-carbonsäure-(2-fluor-4-heptyl-phenylester)
2-Pentyl-1,3-dioxan-5-carbonsäure-(2-fluor-4-cyan-phenylester)
2-Pentyl-1,3-dioxan-5-carbonsäure-(2-fluor-4-ethoxy-phenylester)
2-Pentyl-1,3-dioxan-5-carbonsäure-(2-fluor-4-propoxy-phenylester)
2-Pentyl-1,3-dioxan-5-carbonsäure-(2-fluor-4-butoxy-phenylester)
2-Pentyl-1,3-dioxan-5-carbonsäure-(2-fluor-4-pentoxy-phenylester)
2-Pentyl-1,3-dioxan-5-carbonsäure-(2-fluor-4-hexoxy-phenylester)
2-Pentyl-1,3-dioxan-5-carbonsäure-(2-fluor-4-octoxy-phenylester)
2-Pentyl-1,3-dioxan-5-carbonsäure-(2'-fluor-4'-ethylbiphenyl-4-yl-ester)
2-Pentyl-1,3-dioxan-5-carbonsäure-(2'-fluor-4'-propylbiphenyl-4-yl-ester)
2-Pentyl-1,3-dioxan-5-carbonsäure-(2'-fluor-4'-butylbiphenyl-4-yl-ester)
2-Pentyl-1,3-dioxan-5-carbonsäure-(2'-fluor-4'-pentylbiphenyl-4-yl-ester)

2-Pentyl-1,3-dioxan-5-carbonsäure-(2'-fluor-4'-heptylbiphenyl-4-yl-ester)
2-Pentyl-1,3-dioxan-5-carbonsäure-(2'-fluor-4'-cyanbiphenyl-4-yl-ester)
2-Pentyl-1,3-dioxan-5-carbonsäure-(2'-fluor-4'-ethoxybiphenyl-4-yl-ester)
2-Pentyl-1,3-dioxan-5-carbonsäure-(2'-fluor-4'-propoxybiphenyl-4-yl-ester)
2-Pentyl-1,3-dioxan-5-carbonsäure-(2'-fluor-4'-butoxybiphenyl-4-yl-ester)
2-Pentyl-1,3-dioxan-5-carbonsäure-(2'-fluor-4'-pentoxybiphenyl-4-yl-ester)
2-Pentyl-1,3-dioxan-5-carbonsäure-(2'-fluor-4'-hexoxybiphenyl-4-yl-ester)
2-Pentyl-1,3-dioxan-5-carbonsäure-(2'-fluor-4'-octoxybiphenyl-4-yl-ester)
2-Pentyl-1,3-dioxan-5-carbonsäure-(2-fluor-4'-ethylbiphenyl-4-yl-ester)
2-Pentyl-1,3-dioxan-5-carbonsäure-(2-fluor-4'-propylbiphenyl-4-yl-ester)
2-Pentyl-1,3-dioxan-5-carbonsäure-(2-fluor-4'-butylbiphenyl-4-yl-ester)
2-Pentyl-1,3-dioxan-5-carbonsäure-(2-fluor-4'-pentylbiphenyl-4-yl-ester)
2-Pentyl-1,3-dioxan-5-carbonsäure-(2-fluor-4'-heptylbiphenyl-4-yl-ester)
2-Pentyl-1,3-dioxan-5-carbonsäure-(2-fluor-4'-cyanbiphenyl-4-yl-ester)
2-Pentyl-1,3-dioxan-5-carbonsäure-(2-fluor-4'-ethoxybiphenyl-4-yl-ester)
2-Pentyl-1,3-dioxan-5-carbonsäure-(2-fluor-4'-propoxybiphenyl-4-yl-ester)
2-Pentyl-1,3-dioxan-5-carbonsäure-(2-fluor-4'-butoxybiphenyl-4-yl-ester)
2-Pentyl-1,3-dioxan-5-carbonsäure-(2-fluor-4'-pentoxybiphenyl-4-yl-ester)
2-Pentyl-1,3-dioxan-5-carbonsäure-(2-fluor-4'-hexoxybiphenyl-4-yl-ester)
2-Pentyl-1,3-dioxan-5-carbonsäure-(2-fluor-4'-octoxybiphenyl-4-yl-ester)

**Beispiel 5**

Ein Gemisch von 3,0 g 4-Cyan-3-fluorbenzaldehyd [erhältlich aus 2-Fluor-4-nitrotoluol durch Oxidation zur entsprechenden Benzoesäure, Umwandlung in das entsprechende Nitril, Reduktion zum 2-Fluor-4-aminobenzonitril und Überführung des entsprechenden Diazoniumsalzes mit Formaldoxim in den Aldehyd nach Beech (J. Chem. Soc. 1297 (1954)); das Zielprodukt ist auch erhältlich aus 2-Fluor-4-aminotoluol durch Umwandlung in 3-Fluor-4-methylbenzonitril nach Sandmeyer, Nitrilverseifung, Seitenkettenbromierung des erhältlichen 3-Fluor-4-methyl-benzoesäureethylesters, Umwandlung in den Aldehyd nach Sommelet, Überführung des daraus erhaltenen Oxims in 3-Fluor-4-cyanbenzoesäureethylester, Reduktion zum 3-Fluor-4-cyanbenzylalkohol und nachfolgender Oxidation zum Zielprodukt] und 3,5 g 2-n-Heptylpropandiol-1,3 wird zusammen mit 1 g stark saurem Kationenaustauscher in 100 ml Toluol 8 Stunden am Wasserabscheider erhitzt. Anschließend wird der Katalysator abfiltriert und das Toluol entfernt. Nach üblicher Aufarbeitung erhält man 2-(4-Cyan-3-fluorphenyl)-5-n-heptyl-1,3-dioxan.

Analog werden hergestellt:

2-(4-Cyan-3-fluorphenyl)-5-methyl-1,3-dioxan
2-(4-Cyan-3-fluorphenyl)-5-ethyl-1,3-dioxan
2-(4-Cyan-3-fluorphenyl)-5-propyl-1,3-dioxan
2-(4-Cyan-3-fluorphenyl)-5-butyl-1,3-dioxan
2-(4-Cyan-3-fluorphenyl)-5-pentyl-1,3-dioxan
2-(4-Cyan-3-fluorphenyl)-5-hexyl-1,3-dioxan
2-(4-Cyan-3-fluorphenyl)-5-octyl-1,3-dioxan

**Beispiel 6**

Ein Gemisch von 2,8 g p-(5-n-Pentyl-1,3-dioxan-2-yl)-benzoesäure (DS-OS-3 146 249), 1,6 g 4-Cyan-3-fluorphenol, 2,1 g Dicyclohexylcarbodiimid, 0,1 g 4-Di-methylaminopyridin und 30 ml N,N-Dimethylformamid wird bei 0° C 8 Stunden gerührt. Nach Erwärmen auf Raumtemperatur werden 50 ml Methylenchlorid zugegeben, der angefallene Harnstoff wird abfiltriert. Das Filtrat wird nacheinander mit Salzsäure, Natriumbicarbonat und Wasser gewaschen und wie üblich aufgearbeitet. Man erhält p-(5-n-Pentyl-1,3-dioxan-2-yl)-benzoesäure-(4-cyan-3-fluorphenylester).

Analog werden hergestellt:

p-(5-Methyl-1,3-dioxan-2-yl)-benzoesäure-(4-cyan-3-fluorphenylester)
p-(5-Ethyl-1,3-dioxan-2-yl)-benzoesäure-(4-cyan-3-fluorphenylester)
p-(5-Propyl-1,3-dioxan-2-yl)-benzoesäure-(4-cyan-3-fluorphenylester)
p-(5-Butyl-1,3-dioxan-2-yl)-benzoesäure-(4-cyan-3-fluorphenylester)
p-(5-Hexyl-1,3-dioxan-2-yl)-benzoesäure-(4-cyan-3-fluorphenylester)

p-(5-Heptyl-1,3-dioxan-2-yl)-benzoesäure-(4-cyan-3-fluorphenylester)
p-(5-Octyl-1,3-dioxan-2-yl)-benzoesäure-(4-cyan-3-fluorphenylester)

**Beispiel 7**

Ein Gemisch von 3,0 g 4-Cyan-3-fluorbenzaldehyd und 4,0 g 2-(trans-4-n-Propylcyclohexyl)-propandiol-1,3 wird analog Beispiel 5 umgesetzt. Man erhält 2-(4-Cyan-3-fluorphenyl)-5-(trans-4-n-propylcyclohexyl)-1,3-dioxan.

Analog werden hergestellt:

2-(4-Cyan-3-fluorphenyl)-5-(trans-4-methylcyclohexyl)-1,3-dioxan
2-(4-Cyan-3-fluorphenyl)-5-trans-4-ethylcyclohexyl)-1,3-dioxan
2-(4-Cyan-3-fluorphenyl)-5-(trans-4-butylcyclohexyl)-1,3-dioxan
2-(4-Cyan-3-fluorphenyl)-5-(trans-4-pentylcyclohexyl)-1,3-dioxan
2-(4-Cyan-3-fluorphenyl)-5-(trans-4-hexylcyclohexyl)-1,3-dioxan
2-(4-Cyan-3-fluorphenyl)-5-(trans-4-heptylcyclohexyl)-1,3-dioxan
2-(4-Cyan-3-fluorphenyl)-5-(trans-4-octylcyclohexyl)-1,3-dioxan

**Beispiel 8**

Aus 2-n-Hexyl-1,3-dioxan-5-carbonsäure und 4-Cyan-3-fluorphenol erhält man analog zu Beispiel 6 2-n-Hexyl-1,3-dioxan-5-carbonsäure-(4-cyan-3-fluorphenylester).

Analog werden hergestellt:

2-Methyl-1,3-dioxan-5-carbonsäure-(4-cyan-3-fluor-phenylester)
2-Ethyl-1,3-dioxan-5-carbonsäure-(4-cyan-3-fluor-phenylester)
2-Propyl-1,3-dioxan-5-carbonsäure-(4-cyan-3-fluor-phenylester)
2-Butyl-1,3-dioxan-5-carbonsäure-(4-cyan-3-fluor-phenylester)
2-Pentyl-1,3-dioxan-5-carbonsäure-(4-cyan-3-fluor-phenylester)
2-Heptyl-1,3-dioxan-5-carbonsäure-(4-cyan-3-fluor-phenylester)
2-Octyl-1,3-dioxan-5-carbonsäure-(4-cyan-3-fluor-phenylester)

**Beispiel 9**

Man kocht 2,96 g 3-Fluor-4-(5-Pentyl-1,3-dioxan-2-yl)-benzoesäure (hergestellt aus 2-Fluor-4-bromtoluol durch Umsetzung mit Magnesium nach Grignard und Reaktion mit Kohlendioxid zu 3-Fluor-4-methylbenzoesäure, deren Veresterung mit Ethanol/Schwefelsäure zu 3-Fluor-4-methylbenzoesäureethylester, Bromierung mit Brom oder N-Bromsuccinimid zu 3-Fluor-4-brommethylbenzoesäureethylester, Oxidation mit verdünnter Salpetersäure zu 3-Fluor-terephthalaldehydsäure und deren Ketalisierung mit 2-Pentyl-propan-1,3-diol) 1 Std. mit 2,0 g $SOCl_2$, dampft ein, löst das erhaltene rohe Säurechlorid in 20 ml Toluol, versetzt mit 1,5 ml Pyridin und 1,19 g 4-Cyanphenol und kocht 2 Stunden. Das Gemisch wird mit Wasser versetzt. Man trennt ab, wäscht die organische Phase mit Wasser, trocknet über $Na_2SO_4$, dampft ein und erhält 3-Fluor-4-(5-pentyl-1,3-dioxan-2-yl)-benzoesäure-(4-cyanphenyl)-ester.

Analog werden erhalten:

3-Fluor-4-(5-ethyl-1,3-dioxan-2-yl)-benzoesäure-(4-cyanphenyl)-ester
3-Fluor-4-(5-propyl-1,3-dioxan-2-yl)-benzoesäure-(4-cyanphenyl)-ester
3-Fluor-4-(5-butyl-1,3-dioxan-2-yl)-benzoesäure-(4-cyanphenyl)-ester
3-Fluor-4-(5-hexyl-1,3-dioxan-2-yl)-benzoesäure-(4-cyanphenyl)-ester
3-Fluor-4-(5-heptyl-1,3-dioxan-2-yl)-benzoesäure-(4-cyanphenyl)-ester
3-Fluor-4-(5-octyl-1,3-dioxan-2-yl)-benzoesäure-(4-cyanphenyl)-ester
3-Fluor-4-(5-nonyl-1,3-dioxan-2-yl)-benzoesäure-(4-cyanphenyl)-ester

3-Fluor-4-(5-ethyl-1,3-dioxan-2-yl)-benzoesäure-(4-ethyl-phenyl)-ester
3-Fluor-4-(5-propyl-1,3-dioxan-2-yl)-benzoesäure-(4-ethyl-phenyl)-ester
3-Fluor-4-(5-butyl-1,3-dioxan-2-yl)-benzoesäure-(4-ethyl-phenyl)-ester

3-Fluor-4-(5-pentyl-1,3-dioxan-2-yl)-benzoesäure-(4-ethyl-phenyl)-ester
3-Fluor-4-(5-hexyl-1,3-dioxan-2-yl)-benzoesäure-(4-ethyl-phenyl)-ester
3-Fluor-4-(5-heptyl-1,3-dioxan-2-yl)-benzoesäure-(4-ethyl-phenyl)-ester
3-Fluor-4-(5-octyl-1,3-dioxan-2-yl)-benzoesäure-(4-ethyl-phenyl)-ester
3-Fluor-4-(5-nonyl-1,3-dioxan-2-yl)-benzoesäure-(4-ethyl-phenyl)-ester

3-Fluor-4-(5-ethyl-1,3-dioxan-2-yl)-benzoesäure-(4-propyl-phenyl)-ester
3-Fluor-4-(5-propyl-1,3-dioxan-2-yl)-benzoesäure-(4-propyl-phenyl)-ester
3-Fluor-4-(5-butyl-1,3-dioxan-2-yl)-benzoesäure-(4-propyl-phenyl)-ester
3-Fluor-4-(5-pentyl-1,3-dioxan-2-yl)-benzoesäure-(4-propyl-phenyl)-ester
3-Fluor-4-(5-hexyl-1,3-dioxan-2-yl)-benzoesäure-(4-propyl-phenyl)-ester
3-Fluor-4-(5-heptyl-1,3-dioxan-2-yl)-benzoesäure-(4-propyl-phenyl)-ester
3-Fluor-4-(5-octyl-1,3-dioxan-2-yl)-benzoesäure-(4-propyl-phenyl)-ester
3-Fluor-4-(5-nonyl-1,3-dioxan-2-yl)-benzoesäure-(4-propyl-phenyl)-ester

3-Fluor-4-(5-ethyl-1,3-dioxan-2-yl)-benzoesäure-(4-butyl-phenyl)-ester
3-Fluor-4-(5-propyl-1,3-dioxan-2-yl)-benzoesäure-(4-butyl-phenyl)-ester
3-Fluor-4-(5-butyl-1,3-dioxan-2-yl)-benzoesäure-(4-butyl-phenyl)-ester
3-Fluor-4-(5-pentyl-1,3-dioxan-2-yl)-benzoesäure-(4-butyl-phenyl)-ester
3-Fluor-4-(5-hexyl-1,3-dioxan-2-yl)-benzoesäure-(4-butyl-phenyl)-ester
3-Fluor-4-(5-heptyl-1,3-dioxan-2-yl)-benzoesäure-(4-butyl-phenyl)-ester
3-Fluor-4-(5-octyl-1,3-dioxan-2-yl)-benzoesäure-(4-butyl-phenyl)-ester
3-Fluor-4-(5-nonyl-1,3-dioxan-2-yl)-benzoesäure-(4-butyl-phenyl)-ester

3-Fluor-4-(5-ethyl-1,3-dioxan-2-yl)-benzoesäure-(4-pentyl-phenyl)-ester
3-Fluor-4-(5-propyl-1,3-dioxan-2-yl)-benzoesäure-(4-pentyl-phenyl)-ester
3-Fluor-4-(5-butyl-1,3-dioxan-2-yl)-benzoesäure-(4-pentyl-phenyl)-ester
3-Fluor-4-(5-pentyl-1,3-dioxan-2-yl)-benzoesäure-(4-pentyl-phenyl)-ester
3-Fluor-4-(5-hexyl-1,3-dioxan-2-yl)-benzoesäure-(4-pentyl-phenyl)-ester
3-Fluor-4-(5-heptyl-1,3-dioxan-2-yl)-benzoesäure-(4-pentyl-phenyl)-ester
3-Fluor-4-(5-octyl-1,3-dioxan-2-yl)-benzoesäure-(4-pentyl-phenyl)-ester
3-Fluor-4-(5-nonyl-1,3-dioxan-2-yl)-benzoesäure-(4-pentyl-phenyl)-ester

3-Fluor-4-(5-ethyl-1,3-dioxan-2-yl)-benzoesäure-(4-hexyl-phenyl)-ester
3-Fluor-4-(5-propyl-1,3-dioxan-2-yl)-benzoesäure-(4-hexyl-phenyl)-ester
3-Fluor-4-(5-butyl-1,3-dioxan-2-yl)-benzoesäure-(4-hexyl-phenyl)-ester
3-Fluor-4-(5-pentyl-1,3-dioxan-2-yl)-benzoesäure-(4-hexyl-phenyl)-ester
3-Fluor-4-(5-hexyl-1,3-dioxan-2-yl)-benzoesäure-(4-hexyl-phenyl)-ester
3-Fluor-4-(5-heptyl-1,3-dioxan-2-yl)-benzoesäure-(4-hexyl-phenyl)-ester
3-Fluor-4-(5-octyl-1,3-dioxan-2-yl)-benzoesäure-(4-hexyl-phenyl)-ester
3-Fluor-4-(5-nonyl-1,3-dioxan-2-yl)-benzoesäure-(4-hexyl-phenyl)-ester

3-Fluor-4-(5-ethyl-1,3-dioxan-2-yl)-benzoesäure-(4-heptyl-phenyl)-ester
3-Fluor-4-(5-propyl-1,3-dioxan-2-yl)-benzoesäure-(4-heptyl-phenyl)-ester
3-Fluor-4-(5-butyl-1,3-dioxan-2-yl)-benzoesäure-(4-heptyl-phenyl)-ester
3-Fluor-4-(5-pentyl-1,3-dioxan-2-yl)-benzoesäure-(4-heptyl-phenyl)-ester
3-Fluor-4-(5-hexyl-1,3-dioxan-2-yl)-benzoesäure-(4-heptyl-phenyl)-ester
3-Fluor-4-(5-heptyl-1,3-dioxan-2-yl)-benzoesäure-(4-heptyl-phenyl)-ester
3-Fluor-4-(5-octyl-1,3-dioxan-2-yl)-benzoesäure-(4-heptyl-phenyl)-ester
3-Fluor-4-(5-nonyl-1,3-dioxan-2-yl)-benzoesäure-(4-heptyl-phenyl)-ester

3-Fluor-4-(5-ethyl-1,3-dioxan-2-yl)-benzoesäure-(4-octyl-phenyl)-ester
3-Fluor-4-(5-propyl-1,3-dioxan-2-yl)-benzoesäure-(4-octyl-phenyl)-ester
3-Fluor-4-(5-butyl-1,3-dioxan-2-yl)-benzoesäure-(4-octyl-phenyl)-ester
3-Fluor-4-(5-pentyl-1,3-dioxan-2-yl)-benzoesäure-(4-octyl-phenyl)-ester
3-Fluor-4-(5-hexyl-1,3-dioxan-2-yl)-benzoesäure-(4-octyl-phenyl)-ester
3-Fluor-4-(5-heptyl-1,3-dioxan-2-yl)-benzoesäure-(4-octyl-phenyl)-ester
3-Fluor-4-(5-octyl-1,3-dioxan-2-yl)-benzoesäure-(4-octyl-phenyl)-ester
3-Fluor-4-(5-nonyl-1,3-dioxan-2-yl)-benzoesäure-(4-octyl-phenyl)-ester

3-Fluor-4-(5-ethyl-1,3-dioxan-2-yl)-benzoesäure-(4-nonyl-phenyl)-ester
3-Fluor-4-(5-propyl-1,3-dioxan-2-yl)-benzoesäure-(4-nonyl-phenyl)-ester
3-Fluor-4-(5-butyl-1,3-dioxan-2-yl)-benzoesäure-(4-nonyl-phenyl)-ester
3-Fluor-4-(5-pentyl-1,3-dioxan-2-yl)-benzoesäure-(4-nonyl-phenyl)-ester
3-Fluor-4-(5-hexyl-1,3-dioxan-2-yl)-benzoesäure-(4-nonyl-phenyl)-ester

3-Fluor-4-(5-heptyl-1,3-dioxan-2-yl)-benzoesäure-(4-nonyl-phenyl)-ester
3-Fluor-4-(5-octyl-1,3-dioxan-2-yl)-benzoesäure-(4-nonyl-phenyl)-ester
3-Fluor-4-(5-nonyl-1,3-dioxan-2-yl)-benzoesäure-(4-nonyl-phenyl)-ester

3-Fluor-4-(5-ethyl-1,3-dioxan-2-yl)-benzoesäure-(4-ethoxy-phenyl)-ester
3-Fluor-4-(5-propyl-1,3-dioxan-2-yl)-benzoesäure-(4-ethoxy-phenyl)-ester
3-Fluor-4-(5-butyl-1,3-dioxan-2-yl)-benzoesäure-(4-ethoxy-phenyl)-ester
3-Fluor-4-(5-pentyl-1,3-dioxan-2-yl)-benzoesäure-(4-ethoxy-phenyl)-ester
3-Fluor-4-(5-hexyl-1,3-dioxan-2-yl)-benzoesäure-(4-ethoxy-phenyl)-ester
3-Fluor-4-(5-heptyl-1,3-dioxan-2-yl)-benzoesäure-(4-ethoxy-phenyl)-ester
3-Fluor-4-(5-octyl-1,3-dioxan-2-yl)-benzoesäure-(4-ethoxy-phenyl)-ester
3-Fluor-4-(5-nonyl-1,3-dioxan-2-yl)-benzoesäure-(4-ethoxy-phenyl)-ester

3-Fluor-4-(5-ethyl-1,3-dioxan-2-yl)-benzoesäure-(4-propoxy-phenyl)-ester
3-Fluor-4-(5-propyl-1,3-dioxan-2-yl)-benzoesäure-(4-propoxy-phenyl)-ester
3-Fluor-4-(5-butyl-1,3-dioxan-2-yl)-benzoesäure-(4-propoxy-phenyl)-ester
3-Fluor-4-(5-pentyl-1,3-dioxan-2-yl)-benzoesäure-(4-propoxy-phenyl)-ester
3-Fluor-4-(5-hexyl-1,3-dioxan-2-yl)-benzoesäure-(4-propoxy-phenyl)-ester
3-Fluor-4-(5-heptyl-1,3-dioxan-2-yl)-benzoesäure-(4-propoxy-phenyl)-ester
3-Fluor-4-(5-octyl-1,3-dioxan-2-yl)-benzoesäure-(4-propoxy-phenyl)-ester
3-Fluor-4-(5-nonyl-1,3-dioxan-2-yl)-benzoesäure-(4-propoxy-phenyl)-ester

3-Fluor-4-(5-ethyl-1,3-dioxan-2-yl)-benzoesäure-(4-butoxy-phenyl)-ester
3-Fluor-4-(5-propyl-1,3-dioxan-2-yl)-benzoesäure-(4-butoxy-phenyl)-ester
3-Fluor-4-(5-butyl-1,3-dioxan-2-yl)-benzoesäure-(4-butoxy-phenyl)-ester
3-Fluor-4-(5-pentyl-1,3-dioxan-2-yl)-benzoesäure-(4-butoxy-phenyl)-ester
3-Fluor-4-(5-hexyl-1,3-dioxan-2-yl)-benzoesäure-(4-butoxy-phenyl)-ester
3-Fluor-4-(5-heptyl-1,3-dioxan-2-yl)-benzoesäure-(4-butoxy-phenyl)-ester
3-Fluor-4-(5-octyl-1,3-dioxan-2-yl)-benzoesäure-(4-butoxy-phenyl)-ester
3-Fluor-4-(5-nonyl-1,3-dioxan-2-yl)-benzoesäure-(4-butoxy-phenyl)-ester

3-Fluor-4-(5-ethyl-1,3-dioxan-2-yl)-benzoesäure-(4-pentoxy-phenyl)-ester
3-Fluor-4-(5-propyl-1,3-dioxan-2-yl)-benzoesäure-(4-pentoxy-phenyl)-ester
3-Fluor-4-(5-butyl-1,3-dioxan-2-yl)-benzoesäure-(4-pentoxy-phenyl)-ester
3-Fluor-4-(5-pentyl-1,3-dioxan-2-yl)-benzoesäure-(4-pentoxy-phenyl)-ester
3-Fluor-4-(5-hexyl-1,3-dioxan-2-yl)-benzoesäure-(4-pentoxy-phenyl)-ester
3-Fluor-4-(5-heptyl-1,3-dioxan-2-yl)-benzoesäure-(4-pentoxy-phenyl)-ester
3-Fluor-4-(5-octyl-1,3-dioxan-2-yl)-benzoesäure-(4-pentoxy-phenyl)-ester
3-Fluor-4-(5-nonyl-1,3-dioxan-2-yl)-benzoesäure-(4-pentoxy-phenyl)-ester

3-Fluor-4-(5-ethyl-1,3-dioxan-2-yl)-benzoesäure-(4-hexoxy-phenyl)-ester
3-Fluor-4-(5-propyl-1,3-dioxan-2-yl)-benzoesäure-(4-hexoxy-phenyl)-ester
3-Fluor-4-(5-butyl-1,3-dioxan-2-yl)-benzoesäure-(4-hexoxy-phenyl)-ester
3-Fluor-4-(5-pentyl-1,3-dioxan-2-yl)-benzoesäure-(4-hexoxy-phenyl)-ester
3-Fluor-4-(5-hexyl-1,3-dioxan-2-yl)-benzoesäure-(4-hexoxy-phenyl)-ester
3-Fluor-4-(5-heptyl-1,3-dioxan-2-yl)-benzoesäure-(4-hexoxy-phenyl)-ester
3-Fluor-4-(5-octyl-1,3-dioxan-2-yl)-benzoesäure-(4-hexoxy-phenyl)-ester
3-Fluor-4-(5-nonyl-1,3-dioxan-2-yl)-benzoesäure-(4-hexoxy-phenyl)-ester

3-Fluor-4-(5-ethyl-1,3-dioxan-2-yl)-benzoesäure-(4-heptoxy-phenyl)-ester
3-Fluor-4-(5-propyl-1,3-dioxan-2-yl)-benzoesäure-(4-heptoxy-phenyl)-ester
3-Fluor-4-(5-butyl-1,3-dioxan-2-yl)-benzoesäure-(4-heptoxy-phenyl)-ester
3-Fluor-4-(5-pentyl-1,3-dioxan-2-yl)-benzoesäure-(4-heptoxy-phenyl)-ester
3-Fluor-4-(5-hexyl-1,3-dioxan-2-yl)-benzoesäure-(4-heptoxy-phenyl)-ester
3-Fluor-4-(5-heptyl-1,3-dioxan-2-yl)-benzoesäure-(4-heptoxy-phenyl)-ester
3-Fluor-4-(5-octyl-1,3-dioxan-2-yl)-benzoesäure-(4-heptoxy-phenyl)-ester
3-Fluor-4-(5-nonyl-1,3-dioxan-2-yl)-benzoesäure-(4-heptoxy-phenyl)-ester

3-Fluor-4-(5-ethyl-1,3-dioxan-2-yl)-benzoesäure-(4-octoxy-phenyl)-ester
3-Fluor-4-(5-propyl-1,3-dioxan-2-yl)-benzoesäure-(4-octoxy-phenyl)-ester
3-Fluor-4-(5-butyl-1,3-dioxan-2-yl)-benzoesäure-(4-octoxy-phenyl)-ester
3-Fluor-4-(5-pentyl-1,3-dioxan-2-yl)-benzoesäure-(4-octoxy-phenyl)-ester
3-Fluor-4-(5-hexyl-1,3-dioxan-2-yl)-benzoesäure-(4-octoxy-phenyl)-ester
3-Fluor-4-(5-heptyl-1,3-dioxan-2-yl)-benzoesäure-(4-octoxy-phenyl)-ester
3-Fluor-4-(5-octyl-1,3-dioxan-2-yl)-benzoesäure-(4-octoxy-phenyl)-ester

3-Fluor-4-(5-nonyl-1,3-dioxan-2-yl)-benzoesäure-(4-octoxy-phenyl)-ester

3-Fluor-4-(5-ethyl-1,3-dioxan-2-yl)-benzoesäure-(4-nonoxy-phenyl)-ester
3-Fluor-4-(5-propyl-1,3-dioxan-2-yl)-benzoesäure-(4-nonoxy-phenyl)-ester
3-Fluor-4-(5-butyl-1,3-dioxan-2-yl)-benzoesäure-(4-nonoxy-phenyl)-ester
3-Fluor-4-(5-pentyl-1,3-dioxan-2-yl)-benzoesäure-(4-nonoxy-phenyl)-ester
3-Fluor-4-(5-hexyl-1,3-dioxan-2-yl)-benzoesäure-(4-nonoxy-phenyl)-ester
3-Fluor-4-(5-heptyl-1,3-dioxan-2-yl)-benzoesäure-(4-nonoxy-phenyl)-ester
3-Fluor-4-(5-octyl-1,3-dioxan-2-yl)-benzoesäure-(4-nonoxy-phenyl)-ester
3-Fluor-4-(5-nonyl-1,3-dioxan-2-yl)-benzoesäure-(4-nonoxy-phenyl)-ester

3-Fluor-4-(5-ethyl-1,3-dioxan-2-yl)-benzoesäure-ethylester
3-Fluor-4-(5-propyl-1,3-dioxan-2-yl)-benzoesäure-ethylester
3-Fluor-4-(5-butyl-1,3-dioxan-2-yl)-benzoesäure-ethylester
3-Fluor-4-(5-pentyl-1,3-dioxan-2-yl)-benzoesäure-ethylester
3-Fluor-4-(5-hexyl-1,3-dioxan-2-yl)-benzoesäure-ethylester
3-Fluor-4-(5-heptyl-1,3-dioxan-2-yl)-benzoesäure-ethylester
3-Fluor-4-(5-octyl-1,3-dioxan-2-yl)-benzoesäure-ethylester
3-Fluor-4-(5-nonyl-1,3-dioxan-2-yl)-benzoesäure-ethylester

3-Fluor-4-(5-ethyl-1,3-dioxan-2-yl)-benzoesäure-propylester
3-Fluor-4-(5-propyl-1,3-dioxan-2-yl)-benzoesäure-propylester
3-Fluor-4-(5-butyl-1,3-dioxan-2-yl)-benzoesäure-propylester
3-Fluor-4-(5-pentyl-1,3-dioxan-2-yl)-benzoesäure-propylester
3-Fluor-4-(5-hexyl-1,3-dioxan-2-yl)-benzoesäure-propylester
3-Fluor-4-(5-heptyl-1,3-dioxan-2-yl)-benzoesäure-propylester
3-Fluor-4-(5-octyl-1,3-dioxan-2-yl)-benzoesäure-propylester
3-Fluor-4-(5-nonyl-1,3-dioxan-2-yl)-benzoesäure-propylester

3-Fluor-4-(5-ethyl-1,3-dioxan-2-yl)-benzoesäure-butylester
3-Fluor-4-(5-propyl-1,3-dioxan-2-yl)-benzoesäure-butylester
3-Fluor-4-(5-butyl-1,3-dioxan-2-yl)-benzoesäure-butylester
3-Fluor-4-(5-pentyl-1,3-dioxan-2-yl)-benzoesäure-butylester
3-Fluor-4-(5-hexyl-1,3-dioxan-2-yl)-benzoesäure-butylester
3-Fluor-4-(5-heptyl-1,3-dioxan-2-yl)-benzoesäure-butylester
3-Fluor-4-(5-octyl-1,3-dioxan-2-yl)-benzoesäure-butylester
3-Fluor-4-(5-nonyl-1,3-dioxan-2-yl)-benzoesäure-butylester

3-Fluor-4-(5-ethyl-1,3-dioxan-2-yl)-benzoesäure-pentylester
3-Fluor-4-(5-propyl-1,3-dioxan-2-yl)-benzoesäure-pentylester
3-Fluor-4-(5-butyl-1,3-dioxan-2-yl)-benzoesäure-pentylester
3-Fluor-4-(5-pentyl-1,3-dioxan-2-yl)-benzoesäure-pentylester
3-Fluor-4-(5-hexyl-1,3-dioxan-2-yl)-benzoesäure-pentylester
3-Fluor-4-(5-heptyl-1,3-dioxan-2-yl)-benzoesäure-pentylester
3-Fluor-4-(5-octyl-1,3-dioxan-2-yl)-benzoesäure-pentylester
3-Fluor-4-(5-nonyl-1,3-dioxan-2-yl)-benzoesäure-pentylester

3-Fluor-4-(5-ethyl-1,3-dioxan-2-yl)-benzoesäure-hexylester
3-Fluor-4-(5-propyl-1,3-dioxan-2-yl)-benzoesäure-hexylester
3-Fluor-4-(5-butyl-1,3-dioxan-2-yl)-benzoesäure-hexylester
3-Fluor-4-(5-pentyl-1,3-dioxan-2-yl)-benzoesäure-hexylester
3-Fluor-4-(5-hexyl-1,3-dioxan-2-yl)-benzoesäure-hexylester
3-Fluor-4-(5-heptyl-1,3-dioxan-2-yl)-benzoesäure-hexylester
3-Fluor-4-(5-octyl-1,3-dioxan-2-yl)-benzoesäure-hexylester
3-Fluor-4-(5-nonyl-1,3-dioxan-2-yl)-benzoesäure-hexylester

3-Fluor-4-(5-ethyl-1,3-dioxan-2-yl)-benzoesäure-heptylester
3-Fluor-4-(5-propyl-1,3-dioxan-2-yl)-benzoesäure-heptylester
3-Fluor-4-(5-butyl-1,3-dioxan-2-yl)-benzoesäure-heptylester
3-Fluor-4-(5-pentyl-1,3-dioxan-2-yl)-benzoesäure-heptylester
3-Fluor-4-(5-hexyl-1,3-dioxan-2-yl)-benzoesäure-heptylester
3-Fluor-4-(5-heptyl-1,3-dioxan-2-yl)-benzoesäure-heptylester
3-Fluor-4-(5-octyl-1,3-dioxan-2-yl)-benzoesäure-heptylester
3-Fluor-4-(5-nonyl-1,3-dioxan-2-yl)-benzoesäure-heptylester

3-Fluor-4-(5-ethyl-1,3-dioxan-2-yl)-benzoesäure-octylester
3-Fluor-4-(5-propyl-1,3-dioxan-2-yl)-benzoesäure-octylester
3-Fluor-4-(5-butyl-1,3-dioxan-2-yl)-benzoesäure-octylester
3-Fluor-4-(5-pentyl-1,3-dioxan-2-yl)-benzoesäure-octylester
3-Fluor-4-(5-hexyl-1,3-dioxan-2-yl)-benzoesäure-octylester
3-Fluor-4-(5-heptyl-1,3-dioxan-2-yl)-benzoesäure-octylester
3-Fluor-4-(5-octyl-1,3-dioxan-2-yl)-benzoesäure-octylester
3-Fluor-4-(5-nonyl-1,3-dioxan-2-yl)-benzoesäure-octylester

3-Fluor-4-(5-ethyl-1,3-dioxan-2-yl)-benzoesäure-nonylester
3-Fluor-4-(5-propyl-1,3-dioxan-2-yl)-benzoesäure-nonylester
3-Fluor-4-(5-butyl-1,3-dioxan-2-yl)-benzoesäure-nonylester
3-Fluor-4-(5-pentyl-1,3-dioxan-2-yl)-benzoesäure-nonylester
3-Fluor-4-(5-hexyl-1,3-dioxan-2-yl)-benzoesäure-nonylester
3-Fluor-4-(5-heptyl-1,3-dioxan-2-yl)-benzoesäure-nonylester
3-Fluor-4-(5-octyl-1,3-dioxan-2-yl)-benzoesäure-nonylester
3-Fluor-4-(5-nonyl-1,3-dioxan-2-yl)-benzoesäure-nonylester

**Beispiel 10**

4,44 g 3-Fluor-4-(5-pentyl-1,3-dioxan-2-yl)-benzoesäure wird, wie in Beispiel A) beschrieben, mit $SOCl_2$ in das Säurechlorid überführt. Der nach Entfernen übschüssigen $SOCl_2$ erhaltene Rückstand wird in wäßrige Ammoniaklösung eingetragen und 4 Stunden bei 20°C gerührt. Anschließend saugt man den Niederschlag von Säureamid ab und trocknet. Zur Überführung in das Nitril löst man in 50 ml Pyridin und 10 ml $POCl_3$, rührt 20 Stunden bei 20°C und dampft ein. Man erhält 3-Fluor-4-(5-pentyl-1,3-dioxan-2-yl)-benzonitril.

Analog werden erhalten:

3-Fluor-4-(5-ethyl-1,3-dioxan-2-yl)-benzonitril
3-Fluor-4-(5-propyl-1,3-dioxan-2-yl)-benzonitril
3-Fluor-4-(5-butyl-1,3-dioxan-2-yl)-benzonitril
3-Fluor-4-(5-hexyl-1,3-dioxan-2-yl)-benzonitril
3-Fluor-4-(5-heptyl-1,3-dioxan-2-yl)-benzonitril
3-Fluor-4-(5-ethoxy-1,3-dioxan-2-yl)-benzonitril
3-Fluor-4-(5-propoxy-1,3-dioxan-2-yl)-benzonitril
3-Fluor-4-(5-butoxy-1,3-dioxan-2-yl)-benzonitril
3-Fluor-4-(5-pentoxy-1,3-dioxan-2-yl)-benzonitril
3-Fluor-4-(5-hexoxy-1,3-dioxan-2-yl)-benzonitril
3-Fluor-4-(5-heptoxy-1,3-dioxan-2-yl)-benzonitril

Die folgenden Beispiele betreffen erfindungsgemäße flüssigkristalline Phasen:

**Beispiel A**

Eine flüssigkristalline Phase aus

| | |
|---|---|
| 15 % | p-trans-4-Propylcyclohexyl-benzonitril; |
| 11 % | p-trans-4-Pentylcyclohexyl-benzonitril, |
| 15 % | trans-1-p-Methoxyphenyl-4-propylcylohexan, |
| 5 % | trans-1-p-Ethoxyphenyl-4-propylcyclohexan, |
| 6 % | 4-Cyan-4'-(trans-4-pentylcyclohexyl)-biphenyl, |
| 12 % | 4-Ethyl-4'-(trans-4-propylcylohexyl)-biphenyl, |
| 8 % | 4-Ethyl-4'-(trans-4-pentylcyclohexyl)-biphenyl, |
| 5 % | 4,4'-Bis-(trans-4-propylcyclohexyl)-biphenyl, |
| 6 % | 4-(trans-4-Pentylcyclohexyl)-4'-(trans-4-propylcyclohexyl)-biphenyl, |
| 6 % | 4-(trans-4-Pentylcyclohexyl)-2-fluor-4'-(5-propyl-1,3-dioxan-2-yl)-biphenyl, |
| 4 % | trans-4-Propylcylohexancarbonsäure-(p-methoxy-phenylester), |
| 3 % | trans-4-Propylcyclohexancarbonsäure-(p-ethoxy-phenylester) |
| | und |
| 4 % | trans-4-Butylcylohexancarbonsäure-(p-methoxyphenylester |

hat einen Klärpunkt von 106° und eine optische Anisotropie Δn von +0,13.

**Beispiel B**

Man stellt eine flüssigkristalline Phase her aus

10 % 2-p-Cyanphenyl-5-butyl-1,3-dioxan,
11 % p-trans-4-Pentylcylohexyl-benzonitril,
16 % p-trans-4-Propylcyclohexyl-benzonitril,
8 % 4-Ethyl-4'-cyanbiphenyl
7 % 4-Propyl-4'-cyanbiphenyl,
13 % 4-(trans-4-Pentylcyclohexyl)-2-fluor-4'-(5-propyl-1,3-dioxan-2-yl)-biphenyl,
8 % 4-(trans-4-Propylcyclohexyl)-2-fluor-4'-(5-propyl-1,3-dioxan-2-yl)-biphenyl,
9 % 4-(trans-4-Pentylcyclohexyl)-2-methyl-4'-(5-propyl-1,3-dioxan-2-yl)-biphenyl,
8 % 4-Cyan-4'-(trans-4-propylcyclohexyl)-biphenyl
und
10 % 4,4'-Bis-(trans-4-propylcyclohexyl)-biphenyl.

**Beispiel C**

Man stellt eine flüssigkristalline Phase her aus

16 % p-trans-4-Propylcyclohexyl-benzonitril,
12 % p-trans-4-Pentylcyclohexyl-benzonitril,
15 % trans-1-p-Methoxyphenyl-4-propylcylohexan,
5 % trans-1-p-Ethoxyphenyl-4-propylcyclohexan,
7 % 4-Cyan-4'-(trans-4-pentylcyclohexyl)-biphenyl,
13 % 4-Ethyl-4'-(trans-4-propylcylohexyl)-biphenyl,
8 % 4-Ethyl-4'-(trans-4-pentylcyclohexyl)-biphenyl,
5 % 4,4'-Bis-(trans-4-propylcyclohexyl)-biphenyl,
7 % 4-(trans-4-Pentylcyclohexyl)-4'-(trans-4-propylcyclohexyl)-biphenyl,
0,5 % 4-(trans-4-Pentylcyclohexyl)-2-fluor-4'-(5-propyl-1,3-dioxan-2-yl)-biphenyl,
4,5 % trans-4-Propylcylohexancarbonsäure-(p-methoxy-phenylester),
3 % trans-4-Propylcyclohexancarbonsäure-(p-ethoxyphenylester)
und
4 % trans-4-Butylcylohexancarbonsäure-(p-methoxyphenylester.

## Patentansprüche

1. Verbindungen der Formel I

$R^1$-$A^1$-$Z^1$-$A^2$-$R^2$ I

worin

| | |
|---|---|
| $R^1$ und $R^2$ | jeweils H, eine Alkyl-, Alkoxy-, Alkanoyl-, Alkanoyloxy- oder Alkoxycarbonyl-Gruppe mit 1 - 10 C-Atomen, F, Cl, Br, CN oder $R^3$-$A^3$-$Z^2$-, |
| $A^1$ | -A-, -$A^4$-A- oder -A-$A^4$-, |
| A | eine 1,3-Dioxan-2,5-diyl- oder eine 1,3-Dithian-2,5-diyl-gruppe, |
| $A^2$, $A^3$ und $A^4$ | jeweils eine 1,4-Cyclohexylen-, 1,3-Dioxan-2,5-diyl-, 1,3-Dithian-2,5-diyl-, Piperidin-1,4-diyl- oder 1,4-Bicyclo(2,2,2)octylen-Gruppe oder eine unsubstituierte oder durch ein F-Atom oder eine CN-Gruppe substituierte 1,4-Phenylen-, Pyrimidin-2,5-diyl- oder Pyridazin-3,6-diyl-Gruppe, |
| $Z^1$ und $Z^2$ | jeweils -CO-O-, -O-CO-, -$OCH_2$-, -$CH_2O$ oder eine Einfachbindung, und |
| $R^3$ | H, eine Alkyl-, Alkoxy-, Alkanoyl-, Alkanoyloxy- oder Alkoxycarbonyl-Gruppe mit jeweils 1 - 10 C-Atomen, F, Cl, Br oder CN bedeutet, mit der Maßgabe, daß |

(a) mindestens eine Gruppe $A^2$, $A^3$ und/oder $A^4$ eine durch ein F-Atom oder eine CN-Gruppe substituierte 1,4-Phenylen-, Pyrimidin-2,4-diyl oder Pyridazin-3,6-diyl-Gruppe bedeutet und

(b) 1,3-Dioxan-2,5-diyl- und 1,3-Dithian-2,5-diyl-Gruppen nicht in 2-Stellung und Piperidin-1,4-diyl-Gruppen nicht in 1-Stellung durch Alkoxy, Alkanoyl, Alkanoyloxy, Alkoxycarbonyl, F, Cl, Br,

CN oder $R^3$-$A^3$-$Z^2$ substituiert sind,

sowie Verbindungen der Formeln

| | |
|---|---|
| $R^1$-Dio-Phe-$CH_2CH_2$-Phe-$R^2$ | I4 |
| $R^1$-Dio-Phe-$CH_2CH_2$-Cy-$R^2$ | I5 |
| $R^1$-Dio-Phe-$CH_2CH_2$-Phe-Cy-$R^3$ | I6 |
| $R^1$-Dio-Phe-$CH_2CH_2$-Phe-Dio-$R^3$ | I7 |
| $R^1$-Dio-$CH_2CH_2$-Phe-Phe-$R^2$ | I17 |
| $R^1$-Dio-$CH_2CH_2$-Phe-Phe-Cy-$R^2$ | I18 |
| $R^1$-Dio-$CH_2CH_2$-Phe-Cy-$R^2$ | I19 |

worin

Dio eine 1,3-Dioxan-2,5-diylgruppe
Phe eine 1,4-Phenylengruppe
Cy eine 1,4-Cyclohexylengruppe
und
$R^1$, $R^2$ und $R^3$ jeweils H, eine Alkyl-, Alkoxy-, Alkanoyl-, Alkanoyloxy- oder Alkoxycarbonyl-Gruppe mit 1- 10 C-atomen, F, Cl, Br oder CN

bedeutet und mindestens eine Gruppe Phe eine durch ein F-Atom oder eine CN-Gruppe substituierte 1,4-Phenylen-Gruppe bedeutet.

2. Verfahren zur Herstellung von Verbindungen nach Anspruch 1, dadurch gekennzeichnet, daß man einen entsprechenden Aldehyd mit einem entsprechenden Diol bzw. Dithiol umsetzt,

oder daß man eine Verbindung, die sonst einer Verbindung nach Anspruch 1 entspricht, aber an Stelle von H-Atomen eine oder mehrere reduzierbare Gruppen und/oder C-C-Bindungen enthält, mit einem reduzierenden Mittel behandelt,

oder daß man zur Herstellung von Estern nach Anspruch 1 (worin $Z^1$ und/oder $Z^2$ -CO-O- oder -O-CO- bedeuten und/oder $R^1$ und/oder $R^2$ und/oder $R^3$ eine Carboxylgruppe enthalten) eine entsprechende Carbonsäure oder eines ihrer reaktionsfähigen Derivate mit einem entsprechenden Alkohol oder einem seiner reaktionsfähigen Derivate umsetzt,

oder daß man zur Herstellung von Nitrilen nach Anspruch 1 (worin $R^1$ und/oder $R^2$ und/oder $R^3$ CN bedeuten und/oder worin $A^2$ und/oder $A^3$ und/oder $A^4$ durch eine CN-Gruppe substituiert ist) ein entsprechendes Carbonsäureamid dehydratisiert oder ein entsprechendes Carbonsäurehalogenid mit Sulfamid umsetzt,

oder daß man zur Herstellung von Ethern nach Anspruch 1 (worin $R^1$ und/oder $R^2$ eine Alkoxygruppe bedeuten und/oder $Z^1$ und/oder $Z^2$ eine -$OCH_2$- oder -$CH_2O$-Gruppe sind) eine entsprechende Hydroxyverbindung verethert,

und/oder daß man gegebenenfalls eine entsprechende Chlor- oder Bromverbindung (worin $R^1$ und/oder $R^2$ und/oder $R^3$ Cl oder Br bedeuten und/oder worin $A^2$ und/oder $A^3$ und/oder $A^4$ durch ein Chlor- oder Bromatom substituiert ist) mit einem Cyanid umsetzt,

und/oder daß man gegebenenfalls eine Base nach Anspruch 1 durch Behandeln mit einer Säure in eines ihrer Säureadditionssalze umwandelt,

oder daß man gegebenenfalls eine Verbindung nach Anspruch 1 aus einem ihrer Säureadditionssalze durch Behandeln mit einer Base freisetzt.

3. Verwendung der Verbindungen nach Anspruch 1 als Komponenten flüssigkristalliner Phasen.

4. Flüssigkristalline Phase mit mindestens zwei Komponenten, dadurch gekennzeichnet, daß mindestens eine Komponente eine Verbindung nach Anspruch 1 ist.

5. Flüssigkristallanzeigeelement, dadurch gekennzeichnet, daß es eine flüssigkristalline Phase nach Anspruch 4 enthält.

6. Elektrooptisches Anzeigeelement nach Anspruch 5, dadurch gekennzeichnet, daß es als Dielektrikum eine flüssigkristalline Phase nach Anspruch 4 enthält.

## Claims

1. Compounds of the formula I

$R^1$-$A^1$-$Z^1$-$A^2$-$R^2$ I

wherein

$R^1$ and $R^2$ are each H, an alkyl, alkoxy, alkanoyl, alkanoyloxy or alkoxycarbonyl group with in each case 1 - 10 C atoms, F, Cl, Br, CN or $R^3$-$A^3$-$Z^2$,

| | |
|---|---|
| $A^1$ | is -A-, $-A^4$-A- or $-A-A^4-$, |
| A | is a 1,3-dioxane-2,5-diyl or a 1,3-dithiane-2,5-diyl group, |
| $A^2$, $A^3$ and $A^4$ | are each a 1,4-cyclohexylene, 1,3-dioxane-2,5-diyl, 1,3-dithiane-2,5-diyl, piperidine-1,4-diyl or 1,4-bicyclo(2,2,2)octylene group, or a 1,4-phenylene, pyrimidine-2,5-diyl or pyridazine-3,6-diyl group which is unsubstituted or substituted by one F atom or one CN group, |
| $Z^1$ and $Z^2$ | are each -CO-O-, -O-CO- $-CH_2CH_2-$, $-OCH_2-$, $-CH_2O$ or a single bond and |
| $R^3$ | is H, an alkyl, alkoxy, alkanoyl, alkanoyloxy or alkoxycarbonyl group with in each case 1 - 10 C atoms, F, Cl, Br or CN, with the proviso that |

(a) at least one group $A^2$, $A^3$ and/or $A^4$ is a 1,4-phenylene, pyrimidine-2,5-diyl or pyridazine-3,6-diyl group which is substituted by one F atom or one CN group and

(b) 1,3-dioxane-2,5-diyl and 1,3-dithiane-2,5-diyl groups are not substituted by alkoxy, alkanoyl, alkanoyloxy, alkoxycarbonyl, F, Cl, Br, CN or $R^3$-$A^3$-$Z^2$ in the 2-position and piperidine-1,4-diyl groups are not substituted by these radicals in the 1-position

and compounds of the formulae

| | |
|---|---|
| $R^1$-Dio-Phe-$CH_2CH_2$-Phe-$R^2$ | I4 |
| $R^1$-Dio-Phe-$CH_2CH_2$-Cy-$R^2$ | I5 |
| $R^1$-Dio-Phe-$CH_2CH_2$-Phe-Cy-$R^3$ | I6 |
| $R^1$-Dio-Phe-$CH_2CH_2$-Phe-Dio-$R^3$ | I7 |
| $R^1$-Dio-$CH_2CH_2$-Phe-Phe-$R^2$ | I17 |
| $R^1$-Dio-$CH_2CH_2$-Phe-Phe-Cy-$R^2$ | I18 |
| $R^1$-Dio-$CH_2CH_2$-Phe-Cy-$R^2$ | I19 |

wherein

| | |
|---|---|
| Dio | is a 1,3-dioxane-2,5-diyl group |
| Phe | is a 1,4-phenylene group |
| Cy | is a 1,4-cyclohexylene group |
| and | |
| $R^1$, $R^2$ and $R^3$ | are each H, an alkyl, alkoxy, alkanoyl, alkanoyloxy or alkoxycarbonyl group with 1 - 10 C atoms, F, Cl, Br or CN |

and at least one group Phe is a 1,4-phenylene group which is substituted by one F atom or one CN group.

2. Process for the preparation of compounds according to Claim 1, characterised in that a corresponding aldehyde is reacted with a corresponding diol or dithiol, or in that a compound which otherwise corresponds to a compound according to Claim 1 but contains one or more reducible groups and/or C-C bonds instead of H atoms, is treated with a reducing agent, or in that, to prepare esters according to claim 1 (wherein $Z^1$ and/or $Z^2$ are -CO-O- or -O-CO- and/or $R^1$ and/or $R^2$ and/or $R^3$ contain a carboxyl group), a corresponding carboxylic acid or one of its reactive derivatives is reacted with a corresponding alcohol or one of its reactive derivatives, or in that, to prepare nitriles according to claim 1 (wherein $R^1$ and/or $R^2$ and/or $R^3$ are CN and/or wherein $A^2$ and/or $A^3$ and/or $A^4$ are substituted by one CN group), a corresponding carboxylic acid amide is dehydrated or a corresponding carboxylic acid halide is reacted with sulfamide, or in that, to prepare ethers according to claim 1 (wherein $R^1$ and/or $R^2$ are an alkoxy group and/or $Z^1$ and/or $Z^2$ are a $-OCH_2-$ or $-CH_2O-$ group), a corresponding hydroxy compound is etherified, and/or in that, if appropriate, a corresponding chlorine or bromine compound (wherein $R^1$ and/or $R^2$ and/or $R^3$ are Cl or Br and/or wherein $A^2$ and/or $A^3$ and/or $A^4$ are substituted by one chlorine or bromine atom) is reacted with a cyanide, and/or in that, if appropriate, a base according to Claim 1 is converted into one of its acid addition salts by treatment with an acid, or in that, if appropriate, a compound according to claim 1 is liberated from one of its acid addition salts by treatment with a base.

3. Use of the compounds according to Claim 1 as components of liquid crystal phases.

4. Liquid crystal phase with at least two components, characterised in that at least one component is a compound according to Claim 1.

5. Liquid crystal display element, characterised in that it contains a liquid crystal phase according to Claim 4.

6. Electrooptical display element according to Claim 5, characterised in that it contains a liquid crystal phase according to Claim 4 as the dielectric.

## Revendications

1. Composés de formule I

$$R^1\text{-}A^1\text{-}Z^1\text{-}A^2\text{-}R^2 \qquad I$$

dans laquelle

| | |
|---|---|
| $R^1$ et $R^2$ | représentent chacun, un H, un groupe alkyle, alcoxy, alcanoyl, alcanoyloxy ou alcoxycarbonyle, ayant chacun de 1 à 10 atomes de carbone, un F, Cl, Br, CN ou $R^3$-$A^3$-$Z^2$-, |
| $A^1$ | est -A-, -$A^4$-A- ou -A-$A^4$-, |
| A | est un groupe 1,3-dioxanne-2,5-diyle ou un groupe 1,3-dithiane-2,5-diyle, |
| $A^2$, $A^3$ et $A^4$ | représentent chacun un groupe 1,4-cyclohexylène, 1,3-dioxanne-2,5-diyle, 1,3-dithiane-2,5-diyle-, pipéridine-1,4-diyle ou 1,4-bicyclo(2,2,2)-octylène ou un groupe 1,4-phénylène, pyrimidine-2,5-diyle- ou pyridazine-3,6-diyle non substitué ou substitué par un atome F ou un groupe CN, |
| $Z^1$ et $Z^2$ | représentent chacun -CO-O-, -O-CO-, -$OCH_2$-, -$CH_2O$ ou une liaison simple, et |
| $R^3$ | représente un H, un groupe alkyle, alcoxy, alcanoyle, alcanoyloxy ou alcoxycarbonyle ayant chacun de 1 à 10 atomes de carbone, un F, Cl, Br ou CN, à la condition que |

(a) au moins un groupe $A^2$, $A^3$ et/ou $A^4$ represente un groupe 1,4-phénylène, pyrimidine-2,5-diyle ou pyridazine-3,6-diyle substitué par un atome F ou un groupe CN, et que

(b) les groupes 1,3-dioxanne-2,5-diyle et 1,3-dithiane-2,5-diyle ne sont pas substitués en position 2 et le groupe pipéridine-1,4-diyle n'est pas substitué en position 1 par un alcoxy, alcanoyle, alcanoyloxy, alcoxycarbonyle, F, Cl, Br, CN ou $R^3$-$A^3$-$Z^2$-,

ainsi que des composés de formules

| | |
|---|---|
| $R^1$-Dio-Phe-$CH_2CH_2$-Phe-$R^2$ | I4 |
| $R^1$-Dio-Phe-$CH_2CH_2$-Cy-$R^2$ | I5 |
| $R^1$-Dio-Phe-$CH_2CH_2$-Phe-Cy-$R^3$ | I6 |
| $R^1$-Dio-Phe-$CH_2CH_2$-Phe-Dio-$R^3$ | I7 |
| $R^1$-Dio-$CH_2CH_2$-Phe-Phe-$R^2$ | I17 |
| $R^1$-Dio-$CH_2CH_2$-Phe-Phe-Cy-$R^2$ | I18 |
| $R^1$-Dio-$CH_2CH_2$-Phe-Cy-$R^2$ | I19 |

dans lesquelles

| | |
|---|---|
| Dio | est un groupe 1,3-dioxanne-2,5-diyle |
| Phe | est un groupe 1,4-phénylène |
| Cy | est un groupe 1,4-cyclohexylène |
| et | |
| $R^1$, $R^2$ et $R^3$ | représentent chacun un H, un groupe alkyle, alcoxy, alcanoyle, alcanoyloxy, ou alcoxycarbonyle avec de 1 à 10 atomes de carbone, F, Cl, Br ou CN et au moins un groupe 1,4-phénylène substitué par un atome F ou un groupe CN. |

2. Procédé de préparation de composés selon la revendication 1, caractérisé en ce qu'on fait réagir un aldéhyde correspondant avec un diol ou dithiol correspondant,

ou en ce qu'on traite avec un agent réducteur un composé, qui sinon correspond à un composé selon la revendication 1, mais qui contient à la place des atomes H un ou plusieurs groupes pouvant être réduits et/ou des liaisons C-C-,

ou en ce que, pour préparer des esters selon la revendication 1 (dans lesquels $Z^1$ et/ou $Z^2$ représentent -CO-O- ou -O-CO- et/ou $R^1$ et/ou $R^2$ et/ou $R^3$ contiennent un groupe carboxyle), on fait réagir un acide carboxylique correspondant ou un de ses dérivés réactifs avec un alcool correspondant ou un de ses dérivés réactifs,

ou en ce que, pour préparer des nitriles selon la revendication 1 (dans lesquels $R^1$ et/ou $R^2$ et/ou $R^3$ représentent CN et/ou dans lesquels $A^2$ et/ou $A^3$ et/ou $A^4$ est substitué par un groupe -CN), on déshydrate un amide d'acide carboxylique correspondant ou on fait réagir un halogénure d'acide carboxylique correspondant avec un sulfamide,

ou en ce que, pour préparer des éthers selon la revendication 1 (dans lesquels $R^1$ et/ou $R^2$ représentent un groupe alcoxy et/ou $Z^1$ et/ou $Z^2$ sont un groupe -$OCH_2$- ou -$CH_2O$-), on éthère un composé hydroxy correspondant,

et/ou en ce que, le cas échéant, on fait réagir avec un cyanure un composé chloré ou bromé correspondant (dans lequel $R^1$ et/ou $R^2$ et/ou $R^3$ représentent Cl ou Br et/ou dans lequel $A^2$ et/ou $A^3$ et/ou $A^4$ est substitué par un atome de chlore ou de brome),

et/ou en ce que, le cas échéant, on transforme une base selon la revendication 1 en un de ses sels d'addition d'acide, par traitement avec un acide,

ou en ce que, le cas échéant, on libère un composé selon la revendication 1 d'un de ses sels d'addition d'acide par traitement avec une base.

3. Utilisation de composés selon la revendication 1 comme composants de phases de cristaux liquides.

4. Phase de cristaux liquides avec au moins deux composants, caractérisée en ce qu'au moins un composant est un composé selon la revendication 1.

5. Elément d'affichage à cristaux liquides, caractérisé en ce qu'il contient une phase à cristaux liquides selon la revendication 4.

6. Elément d'affichage électrooptique selon la revendication 5, caractérisé en ce qu'il contient comme diélectrique une phase de cristaux liquides selon la revendication 4.